# EUROPEAN PATENT SPECIFICATION

(11) **EP 1 633 192 B1**
(45) Date of publication and mention of the grant of the patent: **05.05.2010**
(21) Application number: 04732131.0
(22) Date of filing: 11.05.2004
(51) Int. Cl.: A01N 47/44, C08L 33/26, C08L 33/24, C08L 33/12, A01N 25/10, A61L 2/16

(54) **ANTIMICROBIAL COMPOSITION COMPRISING A POLYMERIC BIGUANIDE AND A COPOLYMER AND USE THEREOF**
ANTIMIKROBIELLE ZUSAMMENSETZUNG ENTHALTEND EINE POLYMERISCHE BIGUANIDE UND EIN COPOLYMER UND DEREN VERWENDUNG
COMPOSITION ANTIMICROBIENNE CONTENANT UN BIGUANIDE POLYMERIQUE ET UN COPOLYMERE ET UTILISATION ASSOCIEE

(30) Priority: 15.05.2003 GB 0311164; 29.10.2003 GB 0325239
(43) Date of publication of application: 15.03.2006
(73) Proprietor: Arch UK Biocides Limited, Castleford, West Yorkshire WF10 2JT (GB)
(72) Inventor: HODGE, David John, Moreton-on-Lugg, Hereford HR4 3DR (GB); PEARS, David Alan, Poynton, Cheshire SK12 1XU (GB); GERRARD, John Jeffrey, Plas Newton, Chester CH2 1SF (GB); MCGEECHAN, Paula Louise, Ramsbottom, Bury BL0 9HR (GB)
(74) Representative: Bannerman, David Gardner
(86) International application number: PCT/GB2004/001995
(87) International publication number: WO 2004/100663

(56) References cited:
- EP-A- 0 232 006
- WO-A-00/65915
- WO-A-02/28952
- WO-A-99/40791

## Description

The present invention relates to a method for inhibiting the growth of micro-organisms on surfaces by means of a composition comprising a non-ionic vinyl comb type co-polymer and an antimicrobial agent. The antimicrobial agent is controllably released from the vinyl co-polymer over time thereby providing effective anti-microbial control.

Micro-organisms can be found on many inanimate and animate surfaces. The presence of such micro-organisms can result in unhygienic conditions in hospitals and medical environments, kitchens, bathrooms, toilets and in the preparation and packaging of foodstuffs leading to health risks and contamination.

Several antimicrobial agents exist which are effective against many of the virulent forms of micro-organisms found in the food and health-care environments. Unfortunately, the activity of such agents is insufficient in terms of providing a sustained surface hygienic effect. This may be due to the high water solubility and/or lack of substantivity of the antimicrobial agent on a surface which means that the anti-microbial agents is readily displaced. There is therefore a requirement for an antimicrobial agent or an anti-microbial agent in combination with a delivery system which provides a high degree of anti-microbial kill over a sustained period of time.

The literature describes various cases where micro-organisms and in particular bacterial fouling may cause damage or lead to contamination of surfaces including for example swimming pools, industrial pipes, architectural structures, ships hulls, hospital theatres, teeth and kitchen surfaces. Indeed, there have been many attempts and approaches to overcome the micro-biological problems associated especially with bacterial growth on inanimate and animate surfaces.

European Patent 0182523 describes how certain polymeric compositions are effective at preventing oral bacteria from colonisation on the surface of teeth. In UK Patent 2213721, an anti-staining composition comprising polymers with anti-bacterial agents were shown to be effective against bacteria found in an oral environment.

In European Patent 0232006, coating compositions comprising sulphonated polymers and a microbicide for use in marine environments were shown to have hydrolytic instability. In the above cases, the coating of polymer in an aqueous environment the coating of polymer with or without microbicide was substantially erodable, thereby acting by means of a self-polishing effect, thereby reducing the ability of bacteria to colonise on the surface to be protected.

WO 00/02449 describes a process for the biocidal treatment of surfaces comprising high molecular weight grafted co-polymers.

However, none of the documents above describe an antimicrobial system which has both the ability to eliminate micro-organisms effectively and has a sustained, surface hygienic effect.

The term sustained used hereinafter refers to an anti-microbial agent which is still active even after the surface to which the agent has been applied has been cleansed for example by wiping, rinsing or washing the surface.

Surprisingly we have now found that a combination of certain anti-microbial agents and non-ionic vinyl comb-type co-polymers (referred to hereinafter as non-ionic co-polymers), provides effective and sustained antimicrobial activity when used to inhibit the growth of micro-organisms on surfaces. The present invention therefore provides compositions for the treatment of surfaces based on non-ionic co-polymers with varying functionality in both the backbone and the side chain in combination with an anti-microbial agent, especially a biocide.

Consequently, according to a first aspect of the present invention there is provided a composition comprising:
(i) an anti-microbial agent comprising a polymeric biguanide, alone or in combination with at least one other microbiologically active component selected from the group consisting of quaternary ammonium compounds, monoquaternary heterocyclic amine salts, urea derivatives, amino compounds, imidazole derivatives, nitrile compounds, tin compounds or complexes, isothiazolin-3-ones, thiazole derivatives, nitro compounds, iodine compounds, aldehyde release agents, thiones, triazine derivatives, oxazolidine and derivatives thereof, furan and derivatives thereof, carboxylic acids and the salts and esters thereof, phenol and derivatives thereof, sulphone derivatives, imides, thioamides, 2-mercapto-pyridine-N-oxide, azole fungicides, strobilurins, amides, carbamates, pyridine derivatives, compounds with active halogen groups, and organometallic compounds ; and
(ii) a non-ionic co-polymer of Formula (1) 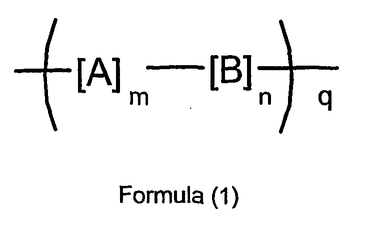
wherein :
[A] is of Formula (9), 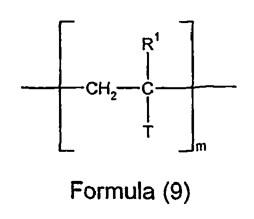
[B] is of Formula (10), 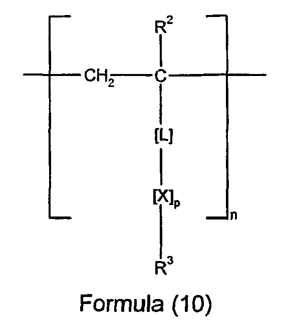 and [X] is of Formula (11), 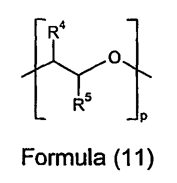
whereas [A] and [B] may occur in any order;
- T: is an optionally substituted substituent;
- L: is an optionally substituted linking group;
- R¹, R² and R³: are each independently H, optionally substituted C₁₋₂₀-alkyl or optionally substituted C₃₋₂₀-cycloalkyl;
- R⁴ and R⁵: are each independently H or C₁₋₄alkyl;
- q: is 15 to 1000;
- p: is 3 to 50; and
the molar ratio of [A] to [B] (m:n), is 1:10 to 10:1;
provided that R¹, R², R³, T and L do not contain an ionisable group and provided that at least one of R⁴ and R⁵ is H.

A preferred anti-microbial agent for use in the composition according to the first aspect of the present invention is an anti-bacterial agent, more preferably a polymeric biguanide.

### Polymeric Biguanide

Preferably the polymeric biguanide comprises at least two biguanide units of Formula (2): 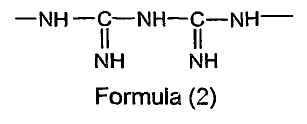 linked by a bridging group which contains at least one methylene group. The bridging group preferably includes a polymethylene chain, optionally incorporating or substituted by one or more hetero atoms such as oxygen, sulphur or nitrogen. The bridging group may include one or more cyclic moieties which may be saturated or unsaturated. Preferably, the bridging group is such that there are at least three, and especially at least four, carbon atoms directly interposed between two adjacent biguanide units of Formula (2). Preferably, there are not greater than ten and especially not greater than eight carbon atoms interposed between two adjacent biguanide units of Formula (2).

The polymeric biguanide may be terminated by any suitable group, such as a hydrocarbyl, substituted hydrocarbyl or an amine group or a cyanoguanidine group of the Formula (3):

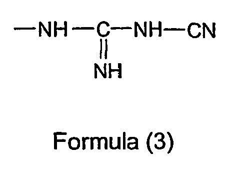

When the terminating group is hydrocarbyl, it is preferably alkyl, cycloalkyl, aryl or aralkyl. When the hydrocarbyl group is alkyl it may be linear or branched but is preferably linear.

Preferred alkyl groups include C₁₋₈-alkyl. Examples of preferred alkyl groups include for example methyl, ethyl, n-propyl, isopropyl, n-pentyl, n-butyl, isobutyl, *tert*-butyl and n-octyl.

When the hydrocarbyl group is cycloalkyl, it is preferably cyclopropyl, cyclopentyl or cyclohexyl. When the hydrocarbyl group is aralkyl, it preferably contains from 1 to 6, more preferably 1 or 2 carbon atoms in the alkylene group attaching the aryl group to the biguanide. Preferred aralkyl groups include benzyl and 2-phenylethyl groups.

Preferred aryl groups include phenyl groups. When the terminating group is substituted hydrocarbyl, the substituent may be any substituent that does not exhibit undesirable adverse effects on the microbiological properties of the polymeric biguanide. Examples of such substituents are aryloxy, alkoxy, acyl, acyloxy, halogen and nitrile.

When the polymeric biguanide contains two biguanide groups of Formula (2) the biguanide is a bisbiguanide. The two biguanide groups are preferably linked through a polymethylene group, especially a hexamethylene group.

The polymeric biguanide preferably contains more than two biguanide units of Formula (1) and is preferably a linear polymeric biguanide which has a recurring polymeric chain represented by Formula (4) or a salt thereof: 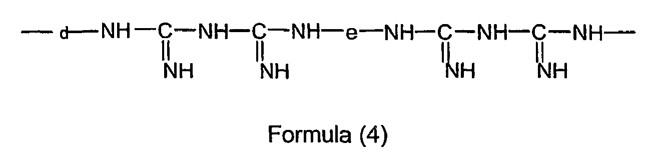 wherein d and e represent bridging groups which may be the same or different and in which together the total of the number of carbon atoms directly interposed between the pairs of nitrogen atoms linked by d plus the number of carbon atoms directly interposed between the pairs of nitrogen atoms linked by e is more than 9 and less than 17.

The bridging groups d and e preferably consist of polymethylene chains, optionally interrupted by hetero atoms, for example, oxygen, sulphur or nitrogen. d and e may also incorporate moieties which may be saturated or unsaturated, in which case the number of carbon atoms directly interposed between the pairs of nitrogen atoms linked by d and e is taken as including that segment of the cyclic group, or groups, which is the shortest. Thus, the number of carbon atoms directly interposed between the nitrogen atoms in the group 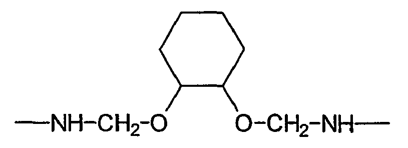 is 4 and not 8.

The linear polymeric biguanides having a recurring polymer unit of Formula (4) are typically obtained as mixtures of polymers in which the polymer chains are of different lengths. Preferably, the number of individual biguanide units of Formulae (5a) and (5b): 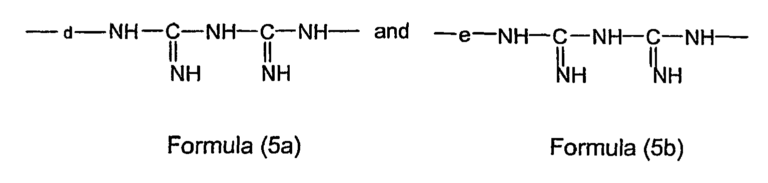 is, together, from 3 to about 80.

The preferred linear polymeric biguanide is a mixture of polymer chains in which d and e are identical and the individual polymer chains, excluding the terminating groups, are of the Formula (6) or a salt thereof: 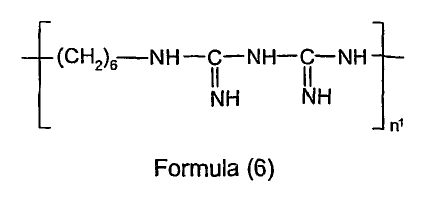 wherein n¹ is from 4 to 20 and especially from 4 to 18. It is especially preferred that the average value of n¹ is about 16. Preferably, the average molecular weight of the polymer in the free base form is from 1100 to 4000.

The linear polymeric biguanides may be prepared by the reaction of a bisdicyandiamide having the Formula (7): 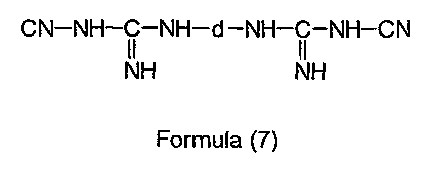 with a diamine H₂N-e-NH₂, wherein d and e have the meanings defined above, or, by the reaction between a diamine salt of dicyanamide having the Formula (8): 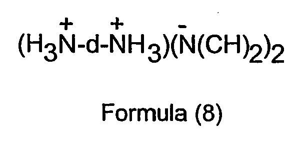 with a diamine H₂N-e-NH₂ wherein d and e have the meanings defined above. These methods of preparation are described in UK specifications numbers 702,268 and 1,152,243 respectively, and any of the polymeric biguanides described therein may be used in the present invention.

As noted hereinbefore, the polymer chains of the linear polymeric biguanides may be terminated either by an amino group or by a cyanoguanidine group of Formula (9):

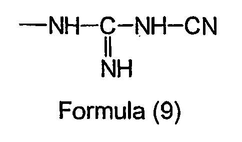

This cyanoguanidine group can hydrolyse during preparation of the linear polymeric biguanide yielding a guanidine end group. The terminating groups may be the same or different on each polymer chain.

A small proportion of a primary amine R-NH₂, where R represents an alkyl group containing from 1 to 18 carbon atoms, may be included with the diamine H₂N-e-NH₂ in the preparation of polymeric biguanides as described above. The primary amine acts as a chain-terminating agent and consequently one or both ends of the polymeric biguanide polymer chains may be terminated by an -NHR group. These -NHR chain-terminated polymeric biguanides may also be used.

The polymeric biguanides readily form salts with both inorganic and organic acids. Preferred salts of the polymeric biguanide are water-soluble.

It is especially preferred that the polymeric biguanide used in accordance with the present invention is a mixture of linear polymers, the individual polymer chains of which, excluding the terminating groups, are represented by Formula (6) in the hydrochloride salt form. This poly(hexamethylenebiguanide) compound is commercially available from Avecia Limited under the trademarks Vantocil^{™}, Cosmocil^{™} and Reputex^{™}.

### Non-Ionic Vinyl Comb Type Co-polymers (Non-ionic co-polymers).

Preferably the non-ionic co-polymers of the present invention are as illustrated in the following Empirical Structural Formula.

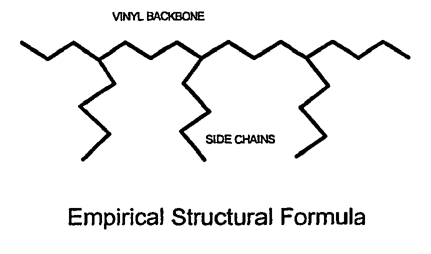

The term non-ionic co-polymer referred to herein is used to describe a co-polymer which can be derived from an addition polymerisation reaction (that is, a free radical initiated process which can be carried out in either an aqueous or non aqueous medium) of two or more olefinically unsaturated monomers. Therefore, the term vinyl monomer used throughout refers to an olefinically unsaturated monomer.

Examples of vinyl monomers which may be used to form non-ionic co-polymers for use in the present invention include but are not limited to styrene, α-methyl styrene, acrylonitrile, methacrylonitrile, vinyl halides such as vinyl chloride, vinylidene halides such as vinylidene chloride, vinyl polyethers of ethylene or propylene oxide such as hydroxypolyethoxy(5) polypropoxy(5)monoallyl ether (BX-AA-E5P5 available from Bimax Chemicals Ltd), vinyl esters such as vinyl acetate, vinyl propionate, vinyl laurate, and vinyl esters of versatic acid (available for example under the trade names VeoVa9 and VeoVa10 from Resolution Performance Products, vinyl ethers of heterocyclic vinyl compounds, alkyl esters of mono-olefinically unsaturated dicarboxylic acids (for example di-n-butyl maleate and di-n-butyl fumarate) and in particular, esters of acrylic acid and methacrylic acid. Vinyl monomers with additional functionality for subsequent crosslinking of the films, such as diacetone acrylamide, aceto acetoxy ethyl methacrylate, glycidyl (meth)acrylate, alkoxy 2-(trimethylsiloxy)ethyl methacrylate, 2-hydroxy ethyl (meth)acrylate, 4-hydroxy butyl (meth)acrylate, 3-hydroxy propyl (meth)acrylate, hydroxy stearyl (meth)acrylate, and 2-hydroxyethyl(meth)acrylate, can also be used.

A particularly preferred non-ionic co-polymer of the present invention is an acrylic co-polymer, derived from esters of acrylic or methacrylic acid.

The non-ionic co-polymers of the present invention comprise at least one polymer which comprises one or more repeating units of Formula (1). 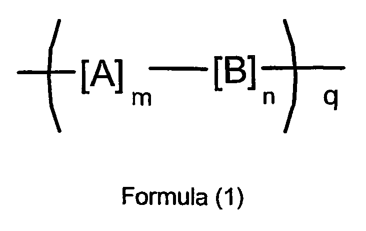 wherein :
[A] is of Formula (9), 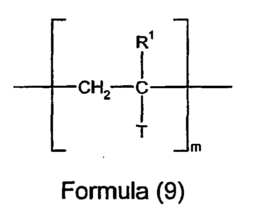
[B] is of Formula (10), 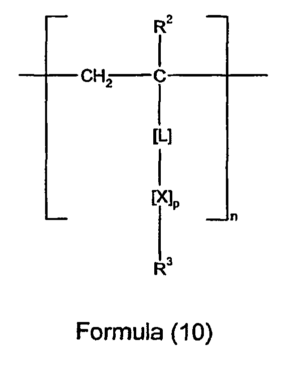 and [X] is of Formula (11), 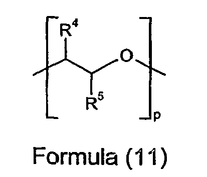
wherein [A] and [B] may occur in any order;
- T: is an optionally substituted substituent;
- L: is an optionally substituted linking group;
- R¹, R² and R³: are each independently H, optionally substituted C₁₋₂₀-alkyl or optionally substituted C₃₋₂₀-cycloalkyl;
- R⁴ and R⁵: are each independently H or C₁₋₄alkyl;
- q: is 15 to 1000;
- p: is 3 to 50; and
the molar ratio of [A] to [B] (m:n), is 1:10 to 10:1;
provided that R¹, R², R³, T and L do not contain an ionisable group and provided that at least one of R⁴ and R⁵ is H.

In Formula (1), [B] provides the non-ionic functionality polyethylene oxide component of the non-ionic co-polymer and [A] is derived from any olefinically unsaturated polymerisable monomer which does not contain an ionisable or ionised functional group.

The molar ratio of [A] to [B] (m:n) is in the range 1:10 to 10:1, more preferably 1:1 to 10:1 and most preferably 2:1 to 4:1.

Preferably the molar ratios of monomers [A] to [B], (m:n) respectively, are also chosen such that the cloud point of the non-ionic co-polymer is greater than 0°C more preferably greater than 15°C and most preferably greater than 25°C. The cloud point value is related to the solubility of the co-polymer in water and refers to the boundary at which liquid-liquid phase separation takes place in a mixture of two or more components indicated by a cloudiness of the solution due to the formation of aggregates that scatter light. The temperature at which a 1% by weight solution of a polymer in distilled water becomes cloudy is the cloud point temperature.

Preferably the polymer comprises 10 to 90% by weight polyethylene oxide provided by [B], more preferably 40 to 85% by weight and most preferably 40 to 75% by weight of [B]. The exact level of polyethylene oxide introduced by [B] required to achieve a cloud point in the preferred range depends on a number of factors for example:
(i) the level and hydrophobicity of [A] in the non-ionic co-polymer.
(ii) the composition of [B] as defined by R², R³, R⁴, R⁵ and the value of p in Formula (11), and
(iii) the presence of organics or electrolytes in solution.

It is preferred that the anti-microbial agent/non-ionic co-polymer compositions of the present invention form a clear solution, that is the cloud point of the non-ionic co-polymer in the presence of anti-microbial agent, for example poly(hexamethylene biguanide) (PHMB) is above 15°C and more preferably above 25°C.

The value of p is 3 to 50, preferably 3 to 40 and most preferably 3 to 25. The value of q is 15 to 1000 most preferably 20 to 400.

R¹, R² and R³ are each independently H, optionally substituted C₁₋₂₀-alkyl or C₃₋₂₀-cycloalkyl. Preferably R¹, R² and R³ are H, C₁₋₁₀-alkyl or C₃₋₈-cycloalkyl. Most preferably R¹ and R² are independently H or CH₃. Most preferably R³ is H or C₁₋₆-alkyl and especially H or CH₃.

R⁴ and R⁵ in repeating units of [X], may be the same or different, and are each independently H or C₁₋₄-alkyl so long as at least one of R⁴ and R⁵ is H. Preferably one of R⁴ and R⁵ is H when the other is -CH₃ or -C₂H₅ with the result that [X] comprises oxyethylene units or a mixture of oxyethylene, oxypropylene and/or oxybutylene units. Most preferably R⁴ and R⁵ are both H, and [X] comprises oxyethylene units.

T is an optionally substituted substituent examples of which include CN, OH, F, Cl, Br, -OR⁶, -C(O)R⁶, -OC(O)R⁶, -C(O)OR⁶, -C(O)NR⁷R⁸ and aryl optionally substituted by -OC(O)R⁶, F, Cl, Br, C₁₋₆-alkyl, -CH₂Cl or -C(O)OR⁶.

R⁶ is C₁₋₁₀-alkyl more preferably C₁₋₈-alkyl for example methyl, ethyl, propyl, butyl, isopropyl, isobutyl or tert-butyl optionally substituted by a ketone, ether, epoxide, silane or ketoester group.

R⁷ and R⁸ are each independently H, C₁₋₈-alkyl or C₃₋₈-cycloalkyl optionally substituted by -OH, ketone or alkyl ether groups, most preferably R⁷ and R⁸ are H, -CH₃ or C₂H₅.

Preferably T is of the formula C(O)OR⁶, -C(O)NR⁷R⁸ or -OC(O)R⁶ and most preferably T is C(O)OR⁶, wherein R⁶, R⁷ and R⁸ are as previously described.

Each L is an optionally substituted linking group which joins [X] to the hydrocarbyl polymer backbone. L can be a variety of linking groups and may be the same or different. Examples of L preferably comprise one or more carbon and/or hetero atoms, for example nitrogen or oxygen. Examples of preferred linking groups represented by L include: 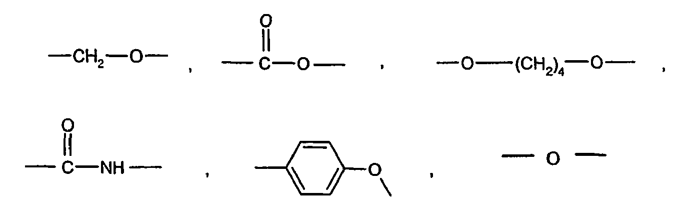 wherein the right hand side of the linking group is attached to [X] and the left hand side of the linking group is attached to the hydrocarbyl backbone.

It is particularly preferred that each L is of formula,

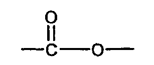

Examples of olefinically unsaturated monomers which may be used for [A] in Formula (1) include: styrene, α-methyl styrene, acrylonitrile, methacrylonitrile, vinyl halides such as vinyl chloride, vinyl esters such as vinyl acetate, vinyl propionate, vinyl laurate, and vinyl esters of versatic acid such as VeoVa™ 9 and VeoVa™ 10 (available from Resolution Performance Products), vinyl ethers of heterocyclic vinyl compounds, in particular, esters of acrylic acid and methacrylic acid. Olefinically unsaturated monomers with additional functionality for subsequent crosslinking and/or adhesion promotion for use in the present invention may also be used, examples of which include diacetone acrylamide, acetoacetoxyethyl-methacrylate and glycidyl methacrylate.

Examples of olefinically unsaturated monomers which may be used for [B] in Formula (1) include: vinyl polyethers of ethylene or propylene oxide, for example hydroxypolyethoxy(5), polypropoxy(5), monoallyl ether (BX-AA-E5P5 available from Bimax Chemicals Ltd), methoxypolyethyleneglycol 350 methacrylate (available from Laporte under the trade name Bisomer MPEG350MA), methoxypolyethyleneglycol 550 methacrylate (available from Laporte under the trade name Bisomer MPEG550 MA), methoxypolyethyleneglycol 350 acrylate, polyethyleneglycol (6) methacrylate PEM6, polyethyleneglycol (6) acrylate PEA6.

Preferred non-ionic co-polymers for use in the present invention are based on acrylic co-polymers, that is co-polymers based on esters of acrylic or methacrylic acid.

Preferably [A] and [B] as defined in Formula (1) for use in the present invention have the Formulae (12) and (13) respectively wherein:
[A] is of Formula (12), 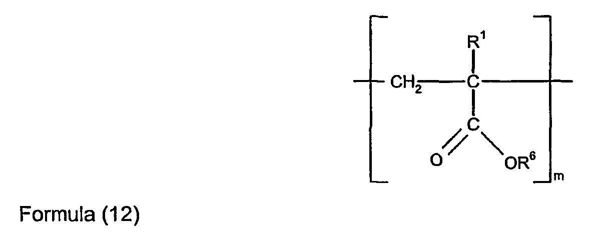
and [B] is of Formula (13),

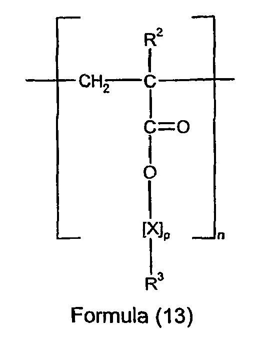

- X: is of Formula (11) as hereinbefore described;
- R¹, R² and R³: are each independently H or CH₃; and
- R⁶: is C₁₋₁₀-alkyl more preferably C₂₋₄-alkyl, optionally substituted by a ketone, ether, -OH, epoxide, silane or ketoester group; and
- n, m and p: are also as hereinbefore defined.

The molar ratios of [A] to [B], (m:n) respectively, are preferably chosen such that the cloud point of the non-ionic co-polymer is greater than 25°C.

Preferred olefinically unsaturated monomers which may be used for [A] of Formula (12) are therefore methyl acrylate, methyl methacrylate, ethyl acrylate, ethyl methacrylate, n-butyl acrylate, n-butyl methacrylate, 2-ethylhexyl acrylate, 2-ethylhexyl methacrylate, isopropyl acrylate, isopropyl methacrylate, n-propyl acrylate, n-propyl methacrylate, t-butyl methacrylate, 2-ethylhexyl methacrylate, isobornyl methacrylate, and cyclohexyl methacrylate and the corresponding acrylates. Methacrylates or acrylates having optional substitution at R⁶ such as for example epoxide, alkyl ether and aryl ether groups, hydroxyalkyl groups for example, hydroxyethyl, hydroxy propyl or hydroxy butyl and modified analogues, may also be employed as part of [A] of Formula (12). Ketofunctional monomers such as the acetoacetoxy esters of hydroxyalkyl acrylates and methacrylates such as acetoacetoxyethyl methacrylate, as well as silane functional monomers such as alkoxysilane alkyl methacrylates may also be used. The advantage of using a functionalised monomer for [A] is that it provides subsequent crosslinkability or adhesion promotion in the resulting polymer.

Examples of the preferred olefinically unsaturated monomers which may be used for [B] in Formula (13) are methoxypolyethyleneglycol 350 methacrylate (available from Laporte under the trade name Bisomer MPEG350 MA), hydroxy polyethyleneglycol 350 methacrylate, methoxypolyethyleneglycol 350 methacrylate (available from Laporte under the trade name Bisomer MPEG550 MA), methoxypolyethyleneglycol 550 acrylate, polyethyleneglycol (6) mono methacrylate PEM6, polyethyleneglycol (6) mono acrylate PEA6.

As illustrated in Figure (1) the non-ionic co-polymers of the present invention comprise a vinyl backbone with pendant side-chains. The non-ionic co-polymers preferably comprises from 10% to 90% by weight of side chain monomer [B] and from 10% to 90% backbone monomer [A].

The non-ionic co-polymers used in the present invention may be prepared by any co-polymerisation method known in the art. Preferably, the co-polymerisation reaction is carried out in water, an organic solvent or a mixture of water and organic solvent using a free radical initiator. Suitable free radical yielding initiators include inorganic peroxides for example potassium, sodium or ammonium persulphate, hydrogen peroxide, or percarbonates; organic peroxides, such as acyl peroxides including for example benzoyl peroxide, alkyl hydroperoxides such as t-butyl hydroperoxide and cumene hydroperoxide; dialkyl peroxides such as di-t-butyl peroxide; peroxy esters such as t-butyl perbenzoate and mixtures thereof may also be used. The peroxy compounds are in some cases advantageously used in combination with suitable reducing agents (redox systems) such as sodium or potassium pyrosulphite or bisulphite, and iso-ascorbic acid. Azo compounds such as azoisobutyronitrile or dimethyl 2,2'-azo bis-isobutylate may also be used. Metal compounds such as iron-ethylenediamine tetracetic acid (EDTA) may also be usefully employed as part of the redox initiator system. Other free radical initiators include: cobalt chelate complexes and particularly Co(II) and Co(III) complexes of porphyrins, dioximes and benzildioxime diboron compounds. It is also possible to use an initiator system partitioning between the aqueous and organic phases, for example a combination of t-butyl hydroperoxide, iso-ascorbic acid and iron-ethylenediamine tetracetic acid. Preferred initiators comprise azo compounds such as azo-iso-butyronitrile and dimethyl 2,2'-azo bis-isobutylate and peroxides such as hydrogen peroxide or benzoyl peroxide. The amount of initiator or initiator system conventionally used is for example within the range of from 0.05 to 6 weight % based on the total amount of vinyl monomers used, more preferably from 0.1 to 3% and most preferably from 0.5 to 2% by weight based on the total amount of vinyl monomers used.

The organic solvent is preferably a polar organic solvent for example a ketone, alcohol or an ether. Examples of suitable polar solvents are methyl ethyl ketone, acetone, methyl isobutylketone, butyl acetate, ethoxyethylacetate, methanol, ethanol, n-propanol, iso-propanol, n-butanol, amyl alcohol, diethylglycol mono-n-butyl ether and butoxyethanol.

Alternatively, the polar organic solvent may be used with a non-polar organic liquid. Suitable non-polar organic solvents include: toluene-xylene mixtures and methylenechloride-dimethylformamide mixtures. Most preferably, the co-polymerisation reaction is carried out in aqueous alcoholic solvents for example, methanol, ethanol, n-propanol, iso-propanol, n-butanol, amyl alcohol, diethylglycol or butoxyethanol, especially aqueous ethanol mixtures.

When prepared by solution polymerisation the number average molecular weight (Mn) of the non-ionic co-polymers is typically in the range 5,000 to 200,000, more preferably 10,000 to 100,000.

The non-ionic co-polymers can also be made by aqueous emulsion or suspension polymerisation (as described in Principles of Polymerisation, G. Odian, Wiley. Interscience 3rd Edition 1991, incorporated herein by reference), in which case the Mn value may be higher and in the range 20,000 to 500,000.

According to the present invention a preferred anti-microbial agent for use in a composition with a non-ionic co-polymer as previously described comprises an antibacterial agent, more preferably a linear polymeric biguanide which is a mixture of polymer chains in which the individual polymer chains, excluding the terminating group are of Formula (6) or a salt thereof as hereinbefore described. A preferred linear polymeric biguanide for use in the present invention is poly(hexamethylenebiguanide) hydrochloride (PHMB) available from Avecia Limited under the trade name Vantocil^{™} IB.

The amount of polymeric biguanide used in the composition of the present invention relative to the amount of non-ionic co-polymer is dependent upon the end use of the composition, the conditions under which it will be stored and the nature of the surface to which the composition is to be applied. The weight ratio of the polymeric biguanide to non-ionic co-polymer in the composition may therefore vary over wide limits for example from 100:1 1 to 1:1000, more preferably from 20:1 to 1:500.

It is especially preferred that the ratio of the polymeric biguanide to non-ionic co-polymer in the antimicrobial composition is from 1:1 to 1:200.

The concentration of polymeric biguanide, for example poly(hexamethylene biguanide) (PHMB) used in the composition of the present invention is preferably in the range of from 0.001 % to 25%, more preferably 0.005% to 10%, and especially 0.01 % to 5%. The pH of the composition is typically chosen so that it is most appropriate for a particular application and is preferably in the range of from 1 to 12, more preferably pH 3 to 9.

The composition of the present invention may also contain other additives depending upon the particular use intended for the composition. Additional components optionally included in the composition may be for example, additional polymeric materials, detergents, botanical extracts, perfumes, fragrances, thickeners, humectants, anti-corrosion agents, surfactants, colourants, chelating agents, buffers, acidity and alkalinity regulators, wetting agents, sequestering agents, hydrotropes, adjuvants, anti-soil agents and enzymes.

For ease of handling and dosing, it is generally convenient to combine the linear polymeric biguanide and non-ionic co-polymer into a formulation with a suitable carrier. The carrier may be a solid but is preferably a liquid and the formulation is preferably a solution, suspension or emulsion of the antimicrobial composition in the liquid.

Whilst water is the preferred carrier for the composition, it is possible that other solvents such as water miscible organic solvents may also be present in the composition. Examples of suitable water-miscible organic solvents include: glycols such as ethylene glycol, propylene glycol, dipropylene glycol, methanol, ethanol, propan-1-ol, propan-2-ol, N-methyl-2-pyrrolidone and lower C₁₋₄-alkyl carbitols such as methyl carbitol. Preferred water-miscible organic solvents are glycols with 2 to 6 carbon atoms, poly-alkylene glycols with 4 to 9 carbon atoms or mono C₁₋₄-alkyl ethers of glycols with 3 to 13 carbon atoms. The most preferred water-miscible organic solvents are propylene glycol, ethyl hexyl glycol or ethanol or C₁₋₆-alkyl esters for example butyl ethyl acetate or pentylacetate.

Therefore, according to a second aspect of the present invention there is provided a formulation comprising:
(i) a linear polymeric biguanide;
(ii) a non-ionic co-polymer; and
(iii) a carrier.

A preferred formulation of the final diluted application liquor according to a second aspect of the invention comprises from 0.01 to 5% by weight polymeric biguanide, more preferably from 0.1 to 1% by weight polymeric biguanide in the form of poly(hexamethylene biguanide) hydrochloride (PHMB). The amount of non-ionic co-polymer in the formulation is preferably from 0.01 to 50% by weight, especially from 0.1 to 25% by weight. The preferred carriers are water or water/alcohol mixtures. The pH of the formulation is typically in the range of from 1 to 12, the pH chosen being that appropriate for the application. Most preferably the pH of the formulation is in the range of from 3 to 9. An especially preferred formulation according to the second aspect of the present invention comprises a diluted application solution containing 0.5% by weight poly(hexamethylene biguanide) hydrochloride (PHMB) and from 2 to 15% by weight non-ionic co-polymer in the form of an aqueous solution.

The formulation may also contain other additives depending upon the particular use intended for the composition. Additional additives optionally included in the formulation are for example those disclosed for use in compositions according to the first aspect of the invention.

During the course of the present studies it has surprisingly been found that when a composition comprising a polymeric biguanide and a non-ionic co-polymer is applied to a surface a sustained anti-microbial effect against a broad range of micro-organisms including gram positive bacteria, gram negative bacteria, pathogenic bacteria, yeasts, fungi and algae is achieved. Therefore according to a further aspect of the present invention there is provided a method of treating a surface which comprises treating the surface with a composition or a formulation as hereinbefore described with reference to the first and second aspects of the present invention.

The preferred biguanide poly(hexamethylene biguanide) hydrochloride, may be the only microbiologically active compound present in the composition or formulation. Alternatively, other microbiologically active compounds may also be present in combination with the polymeric biguanides. Examples of other microbiologically active compounds include for example: quaternary ammonium compounds for example, N,N-diethyl-N-dodecyl-N-benzylammonium chloride, N,N-dimethyl-N-octadecyl-N-(dimethyl benzyl) ammonium chloride, N,N-dimethyl-N,N-didecylammonium chloride, N,N-dimethyl-N,N-didodecylammonium chloride; N,N,N-trimethyl-N-tetradecylammonium chloride, N-benzyl-N,N-dimethyl-N-(C₁₂-C₁₈ alkyl)ammonium chloride, N-(dichlorobenzyl)-N,-N-dimethyl-N-dodecylammonium chloride, N-hexadecylpyridinium chloride, N-hexadecyl pyridinium bromide, N-hexadecyl-N,N,N-trimefihylammonium bromide, N-dodecyl pyridinium chloride, N-dodecylpyridinium bisulphate, N-benzyl-N-dodecyl-N,N-bis(beta-hydroxy-ethyl)ammonium chloride, N-dodecyl-N-benzyl-N,N-dimethylammonium chloride, N-benzyl-N,N-dimethyl-N-(C₁₂-C₁₈ alkyl) ammonium chloride, N-dodecyl-N,N-dimethyl-N-ethylammonium ethylsulphate, N-dodecyl-N,N-dimethyl-N-(1-naphthylmethyl)ammonium chloride, N-hexadecyl-N,N-dimethyl-N-benzylammonium chloride, N-dodecyl-N,N-dimethyl-N-benzylammonium chloride or 1-(3-chloroallyl)-3,5,7-triaza-1-azonia-adamantane chloride, cocoalkylbenzyl-dimethylammonium, tetradecylbenzyldimethyl ammonium chlorides, myristyltrimethyl ammonium or cetyltrimethylammonium bromides, monoquaternary heterocyclic amine salts such as laurylpyridinium, cetylpyridinium or (C₁₂-C₁₄)alkyl benzylimidasolium chlorides; urea derivatives for example, 1,3-bis(hydroxymethyl)-5,5-dimethylhydantoin, bis(hydroxymethyl)urea, 3-(3,4-dichlorophenyl)-1,1-dimethylurea (Diuron), 3-(4-isopropylphenyl)-1,1-dimethylurea, tetrakis(hydroxymethyl)-acetylenediurea, 1-(hydroxymethyl)-5,5-dimethylhydantoin or imidazolidinylurea; amino compounds for example, 1,3-bis(2-ethyl-hexyl)-5-methyl-5-aminohexahydro-pyrimidine, hexamethylenetetramine, 1,3-bis(4-aminophenoxy)propane, dodecylamine or 2-[(hydroxymethyl)-amino]ethanol; imidazole derivatives for example 1[2-(2,4-dichloro-phenyl)-2-(2-propenyloxy)ethyl]-1H-imidazole or 2-(methoxycarbonyl-amino)-benzimidazole (Carbendazim); nitrile compounds for example, 2-bromo-2-bromomethyl-glutaronitrile, 2-chloro-2-chloro-methylglutaro-nitrile, 1,2-dibromo-2,4-dicyanobutane or 2,4,5,6-tetrachloro-1,3-benzenedicarbonitrile (Chlorothalonil); thiocyanate derivatives for example methylene(bis)thiocyanate or 2-(thiocyanomethylthio)-benzothiazole; tin compounds or complexes for example, tributyltinoxide chloride, naphthoate, benzoate or 2-hydroxybenzoate; isothiazolin-3-ones, for example 4,5-trimethylene-4-isothiazolin-3-one, 2-methyl-4,5-trimethylene-4-isothiazolin-3-one, 2-methyl-4-isothiazolin-3-one (MIT), 5-chloro-2-methyl-4-isothiazolin-3-one (CMIT), 2-octylisothiazolin-3-one (OIT) or 4,5-dichloro-2-octyl-4-isothiazolin-3-one (DCOIT); benzisothiazolin-3-one compounds for example 1,2-benzisothiazolin-3-one (BIT), 2-methylbenzisothiazolin-3-one, 2-n-butylbenzisothiazolin-3-one, N-ethyl, N-n-propyl, N-n-pentyl, N-cyclopropyl, N-isobutyl, N-n-hexyl, N-n-octyl, N-n-decyl and N-tert-butyl1,2-benzisothiazolinone; thiazole derivatives for example, 2-(thiocyano methylthio)-benzthiazole or mercaptobenzthiazole; nitro compounds for example, tris(hydroxymethyl)nitromethane, 5-bromo-5-nitro-1,3-dioxane or 2-bromo-2-nitropropane-1,3-diol (Bronopol); iodine compounds, for example tri-iodo allyl alcohol; aldehydes and aldehyde release agents, for example glutaraldehyde (pentanedial), formaldehyde or glyoxal; amides for example chloracetamide, N,N-bis(hydroxymethyl)chloracetamide, N-hydroxymethyl-chloracetamide or dithio-2,2-bis(benzmethylamide); guanidine derivatives for example 1,6-hexamethylene-bis[5-(4-chlorophenyl)biguanide], 1,6-hexamethylene-bis[5-(4-chlorophenyl)guanide], bis(guanidinooctyl)amine triacetate, 1,6-D-(4'-chlorophenyldiguanide)-hexan (Chlorhexidine), polyoxyalkylene-guanidin-hydrochloride, polyhexamethyleneguanidine hydrochloride (PHMG), poly-(2-(2-ethoxy) ethoxyethyl guanidium chloride (PEEG) or dodecyl guanidine hydrochloride; thiones for example 3,5-dimethyltetrahydro-1,3,5-2H-thiodiazine-2-thione; sulphamides, for example N-dimethyl-N'-phenyl-(fluorodichloromethylthio)sulphamide (Preventol A4); triazine derivatives for example hexahydrotriazine, 1,3,5-tri-(hydroxyethyl)-1,3,5-hexahydrotriazine, 6-chloro-2,4-diethyl-amino-s-triazine or 4-cyclopropylamino-2-methylthio-6-t-butylamino-s-triazine (Irgarol); oxazolidine and derivatives thereof for example bis-oxazolidine; furan and derivatives thereof for example 2,5-dihydro-2,5-dialkoxy-2,5-dialkylfuran; carboxylic acids and the salts and esters thereof for example sorbic acid and 4-hydroxybenzoic acid; phenol and derivatives thereof for example 5-chloro-2-(2,4-dichloro-phenoxy)phenol, thio-bis(4-chlorophenol), 2-phenylphenol, 2,4,5-trichloro-2'-hydroxy-diphenylether (Triclosan) and 4-chloro-3,5-dimethyl-phenol (PCMX); sulphone derivatives for example diiodomethyl-paratolylsulphone, 2,3,5,6-tetrachloro-4-(methylsulphonyl)pyridine or hexachlorodimethylsulphone; imides for example, N-(fluorodichloromethylthio)phthalimide (Preventol A3), N-(trichloromethylthio)phthalimide (Folpet) or N-(trichloromethyl)thio-4-cyclohexene-1,2-dicarboxyimide (Captan); thioamides the metal complexes and salts thereof for example dimethyldithiocarbamate, ethylenebisdithiocarbamate, 2-mercapto-pyridine-N-oxide (especially the 2:1 zinc complex and the sodium salt); azole fungicides for example hexaconazole, tebuconazole, propiconazole, etaconazole or tetraconazole; strobilurins, for example methyl-(E)-2-[2-(6-(2-cyanophenoxy)pyrimidin-4-yloxy)phenyl]-3-methoxyacrylate (Azoxystrobin), methyl-(E)-methoxyimino[α-(o-tolyloxy)-o-tolyl]acetate, N-methyl-(E)-methoxyimino[2-(2,5-dimethlyphenoxymethyl)phenyl] acetamide, N-methyl-(E)-2-methoxyimino-2-(2-phenoxyphenyl)acetamide (Metominostrobin) or Trifloxystrobin; amides for example dithio-2,2'-bis(benzmethylamide) (Densil P) or 3,4,4'-Trichlorocarbanilide (Triclocarban); carbamates for example 3-Iodopropargyl-N-butylcarbamate (IPBC), 3-Iododpropargyl-N-phenylcarbamate (IPPC) or Bis-(diemthylthiocarbamoyl)-disulphide (Thiram); pyridine derivatives for example sodium or zinc salt of 2-mercaptopyridine-N-oxide (Sodium or Zinc pyrithione); compounds with activated halogen groups for example tetrachloroisophthalodintril (Chlorthalonil), 1,2-Dibromo-2,4-dicyanobutane (Tektamer 38); organometallic compounds for example 10,10'-Oxybisphenoxyarsine (OBPA).

The amount of further antimicrobial compound(s) in the composition will depend upon the further antimicrobial compound and the surface to be protected.

It is further possible to use combinations of two or more non-ionic co-polymers of Formula (1) as previously described with the anti-microbial compounds as previously described for the compositions or formulations of the present invention for disinfecting surfaces found in for example household, industrial or institutional areas. The treatment can be applied to a wide variety of surfaces as exemplified as follows but not limited thereto. Surface applications include for example, walls, floors, work surfaces, equipment found in domestic, industrial, food processing, sanitary, health and medical environments, skin, synthetic and natural textiles and fibres, stainless steel, polymer and polymeric coatings such as vinyl, polyvinyl chloride, polypropylene and polyethylene, wood, glass, rubber, paint surfaces, stone, marble, grouts, packaging and films.

As hereinbefore described the anti-microbial compositions according to the first and second aspects of the invention significantly reduce the levels of micro-organisms on surfaces treated with the anti-microbial compositions, which activity is sustained over a period of time.

According to a fourth aspect of the present invention there is therefore provided the use of a composition according to the first aspect of the present invention or the use of a formulation according to a second aspect of the present invention for the treatment of surfaces.

It has also been found that the non-ionic co-polymers described above in relation to the present invention may also be used in combination with anti-fungal compounds. It has surprisingly been found that fungicidal compounds are also controllably released from the non-ionic co-polymers over time thereby providing sustained and effective anti-fungal control.

### Fungicides

A wide variety of fungicides can be used in combination with the non-ionic co-polymers described above. Examples of such fungicides include but are not limited to:
methoxyacrylates, for example, methyl (E)-2-2-6-(2-cyanophenoxy)pyrimidin-4-yloxyphenyl-3-methoxyacrylate; carboxamides and acetamides for example, 5,6-dihydro-2-methyl-N-phenyl-1,4-oxathiin-3-carboxamide and 2-cyano-N-[(ethylamino)carbonyl]-2-(methoxyamino)acetamide; aldehydes, for example cinnamaldehyde and 3,5-dichloro-4-hydroxybenzaldehyde; pyrimidines, for example 4-cyclopropyl-6-methyl-N-phenyl-2-pyrimidinamine and 5-butyl-2-ethylamino-6-methylpyrimidin-4-ol; morpholines for example, (E,Z)-4-[3-(4-chlorophenyl)-3-(3,4-dimethoxyphenyl)acryloyl]morpholine and C₁₁₋₄-alkyl-2,6-dimethylmorpholine-homologues such as (Tridemorph) and (±)-cis-4-[3-tert-butylphenyl)-2-methylpropyl]-2,6-dimethylmorpholine (Fenpropimorph); guanidines, for example 1-dodecylguanidine acetate; pyrroles, for example 4-(2,2-difluoro-1,3-bezodioxol-4-yl)-1Hpyrrole-3-carbonitrile; imidazoles and benzimidazoles, for example 1-[2-(2,4-dichlorophenyl)-2-(2-propenyloxy)ethyl]-1H-imidazole, 3-(3,5-dichlorophenyl)-N-(1-methylethyl)-2,4-dioxo-1-imidazolidinecarboxamide, Carbendazim (MBC), Benomyl, Fuberidazole, Thiabendazole, 1-(N-propyl-N-(2-(2,4,6-(trichlorophenoxy)-ethyl)-carbamoyl)-imidazole (prochloraz) and salts thereof; alanine derivatives for example, N-(2,6-dimethylphenyl)-N-(methoxyacetyl)-D-alaninemethyl ester and N-(2,6-dimethylphenyl)-N-(methoxyacetyl)-DL-alaninemethyl ester; triazoles for example, 1-[[2-(2,4-dichlorophenyl)-4-propyl-1,3-dioxolan-2-yl]methyl]-1H-1,2,4-triazole, H-1,2,4-triazole-1-ethanol-alpha-[2-(4-chlorophenyl)-ethyl]-alpha-(1,1-dimethylethyl),1-[2-( 2,4-dichlorophenyl)-1,3-dioxolan-2-yl-methyl]-1H-1,2,4-triazole [azaconazole], 1-(4-chlorophenoxy)-3,3-dimethyl-1-(1H-1,2,4-triazol-1-yl)-2-butanone (triadimefone), β-(4-chlorophenoxy)-α-(1,1-dimethyl-ethyl)-1H-1,2,4-triazole-1- ethanol (triadimenol), α-[2-(4-chlorophenyl)-ethyl]- α-(1,1-dimethylethyl)-1H-1,2, 4-triazole-1-ethanol (tebuconazole), (RS)-2-(2,4-dichlorophenyl)-1-(1H-1,2,4-triazol-2-yl)-hexan-2-ol (hexaconazole), 1-[[2-(2,4-dichlorophenyl)-4-n-propyl-1,3-dioxolan-2-yl]-methyl]-1-H-1,2,4-triazole (propiconazole). Triazole fungicides can be present not only in the form of free bases but also in the form of their metal salt complexes or as acid addition salts, for example salts of metals of main groups II to IV and sub-groups I and II and IV to VII of the periodic table of elements, examples of which may include copper, zinc, manganese, magnesium, tin, iron, calcium, aluminium, lead, chromium, cobalt and nickel. Possible anions of the salts are those which are preferably derived from the following acids: hydrohalic acids, for example, hydrochloric acid, hydrobromic acid, phosphoric acid, nitric acid and sulphuric acid. In cases where the compound has an asymmetric carbon atom, isomers and isomer mixtures are also possible. Further examples of fungicides include: oxazolidines for example, 3-(3,5-dichlorophenyl)-5-methyl-5-vinyl-1,3-oxazolidine-2,4-dione; p-hydroxybenzoates for example, benzoic acid, paramethylbenzoic acid, salicylic acid, dehydroacetic acid and salts thereof; isothiazolinones, for example 2-methylisothiazolin-3-one, 5-chloro-2-methylisothiazolin-3-one, 4,5-dichloro-N-octyl-isothiazolin-3-one, 2-n-octyl-4-isothiazolin-3-one, cyclopentenisothiazolinones; benzisothiazolin-3-one compounds for example 2-methylbenzisothiazolin-3-one, 2-n-butylbenzisothiazolin-3-one N-ethyl, N-n-propyl, N-n-pentyl, N-n-hexyl, N-cyclopropyl, and N-isobutylbenzisothiazolin-3-one; quaternary ammonium compounds for example, cocoalkylbenzyl-dimethylammonium, tetradecylbenzyldimethylammonium chlorides, myristyltrimethyl ammonium, cetyltrimethylammonium bromides, monoquaternary heterocyclic amine salts, laurylpyridinium, cetylpyridinium or (C₁₂-C₁₄)alkyl benzylimidasolium chlorides, benzyldimethyltetradecylammoniumchloride, benzyl-dimethyl-dodecylammoniumchloride, didecyl-dimethyl-ammoniumchloride, alkyl ammonium halides, for example lauryl trimethyl ammonium chloride and dilauryl dimethyl ammonium chloride, alkyl aryl ammonium halides such as octadecyl dimethyl benzyl ammonium bromide, ethyl dimethyl stearyl ammonium chloride, trimethyl stearyl ammonium chloride, trimethyl cetyl ammonium chloride, dimethyl ethyl lauryl ammonium chloride, dimethyl propyl myristyl ammonium chloride, dinonyl dimethyl ammonium chloride, didecyl dimethyl ammonium chloride, diundecyl dimethyl ammonium chloride, didecyl dimethyl ammonium chloride, dinonyly ethyl ammonium chloride, dimethyl ethyl benzyl ammonium chloride, 3-(trimethyxyosilyl) propyldidecylmethyl ammonium chloride, 3-(trimethyoxysilyl) propyloctadecycdimethyl ammonium chloride, dimethyl dioctyl ammonium chloride, didecyl dimethyl ammonium chloride, didodecyl dimethyl ammonium chloride, dimethyl ditetradecyl ammonium chloride, dihexadecyl dimethyl ammonium chloride, dimethyl dioctadecyl ammonium chloride, decyl dimethyl octyl ammonium chloride, dimethyl dodecyloctyl ammonium chloride, benzyl decyl dimethyl ammonium chloride, benzyl dimethyl dodecyl ammonium chloride, benzyl dimethyl tetradecyl ammonium chloride, decyl dimethyl (ethyl benzyl) ammonium chloride, decyl dimethyl (dimethyl benzyl)-ammonium chloride, (chlorobenzyl)-decyl dimethyl ammonium chloride, decyl-(decyl-(dichlorobenzyl)-dimethyl ammonium chloride, benzyl didecyl methyl ammonium chloride, benzyl didocyl methyl ammonium chloride, benzyl ditetradecyl methyl ammonium chloride, and benzyl dodecyl ethyl ammonium chloride; iodopropargyl derivatives for example, 3-iodo-2-propynyl-N-n-butylcarbamate (IPBC), propyl 3-(dimethylamino)propylcarbamate-hydrochlorides, 3-iodo-2-propynyl-N-n-propyl carbamate, 3-iodo-2-propynyl-N-n-hexyl carbamate, 3-iodo-2-propynyl-N-cyclohexylcarbamate, 3-iodo-2-propynyl-N-phenyl carbamate and thiocarbamates for example S-ethyl cyclohexyl(ethyl)thiocarbamate; sulphenamides for example, Dichlofluanid (Euparen), Tolylfluarid (Methyleuparen), Folpet, Fluorfolpet, tetramethyldiuramdisulfides (TMTD) and 2-methylbenzamide-1, 1'disulphide (available as Densil™P from Avecia Ltd); thiocyanates for example, thiocyanatomethylthiobenzothiazole (TCMTB) and methylenbisthiocyanate (MBT); phenols for example, o-phenylphenol, tribromphenol, tetrachlorphenol, pentachlorphenol, 2-phenoxyethnaol 3-methyl-4-chlorphenol, dichlorophen and chlorophen; iododeriatives for example, diiodmethyl-p-arylsulfone and diiodmethyl-p-tolylsulfone; bromoderivatives for example, 2-bromo-2-nitro-1,3-propanediol(Bronopol) and 1,2-dibromo-2,4-dicyanobutane (Tektamer™38); pyridines for example, 1-hydroxy-2-pyridinthione or pyridine-2-thiol-1-oxide (sodium, iron, manganese or zinc salts commercially available under the trademark Sodium Omadine from Arch Chemicals), tetrachlor-4-methylsulphonylpyridine, 2,3,5,6 tetrachloro-4(methyl sulphonyl)pyridine (available from Avecia Limited as Densil^{™} S); metallic soaps for example, tin, copper, zinc-naphthenate, octoate, 2-ethylhexanoate, oleate, -phosphate, benzoate, or oxides for example TBTO, Cu₂O, CuO and ZnO; organic tin-derivatives, for example tributyltin naphthenate or tributyl tinoxide; dialkyldithiocarbamates for example sodium and zinc salts of dialkyldithiocarbamates; nitriles for example 2,4,5,6-tetrachlorisophthalonitrile (Chlorthalonil); benzthiazoles, for example 2-mercaptobenzothiazoles; Dazomet; chinolines for example 8-hydroxyquinoline; Tris-N-(cyclohexyldiazeniumdioxy)-aluminum, N-(cyclohexyldiazeniumdioxy)-tributyl tin or potassium salts and Bis-(N-cyclohexyl)diazinium (-dioxy- copper or aluminum); alkyl esters of parahydroxybenzoic acid particularly the methyl, ethyl, propyl and; 2,4,4'-trichloro-2-hydroxydiphenyl ether (available under the trade name Triclosan) or 4,4'-trichloro-2-hydroxydiphenyl ether available under the tradename Diclosan); formaldehyde release compounds for example hydantoins, N,N"-methylene bis[N'-(hydroxymethyl)-2,5-dioxo-4-imidazolidinyl]urea, Quaternium-15 and 1,3-dimethylol-5,5-dimethylhydantoin (DMDMH), N-(hydroxymethyl)-N-(1,3-dihydroxymethyl-2,5-dioxo-4-imidazolidinyl)-N'-(hydroxymethyl); urea and the cis isomer of 1-(3-chloroallyl)-3,5,7-triaza-1-azoniaadamantane chloride; benzylalcoholmono(poly)hemiformal, oxazolidine, hexahydro-s-triazine and N-methylolchloracetamid; cyclic thiohydroxamic acid compounds for example imidazolidine-2-thione, pyrrolinethione, pyrrolidinethione, isoindolinethione, 3-hydroxy-4-methylthiazol-2(3H)-thione, 3-hydroxy-4-phenylthiazol-2(3H)-thione, 3-hydroxy-4,5,6,7-tetrahydrobenzothiazol -2(3H) -thione, 5,5-dimethyl-1-hydroxy-4-imino-3- phenylimidazolidine-2-thione, 1-hydroxy-4-imino-3-phenyl-2-thiono-1,3-diazaspiro[4,5]-decane, 1-hydroxy-5--methyl-4-phenylimidazoline-2-thione, 4,5-dimethyl-3-hydroxythiazol-2(3H)-thione, 4-ethyl-3-hydroxy-5-methylthiazol-2(3H)-thione, 4-(4-chlorophenyl)-3-hydroxythiazol -2(3H)- thione, 3-hydroxy-5-methyl-4-phenylthiazol-2(3H)-thione, 1-hydroxypyrrolidin-2-thione, 5,5-dimethyl-1-hydroxypyrrolidin-2-thione and 2-hydroxy-2,3-dihyro-1H-isoindol-1-thione.

Preferred antifungal compounds include for example quaternary ammonium compounds, isothiazolinone and benzisothiazolinone compounds, carbamates and pyridine compounds.

Therefore, according to a fifth aspect of the present invention there is provided a composition comprising:
(i) a fungicide; and
(ii) a non-ionic co-polymer of Formula (1) 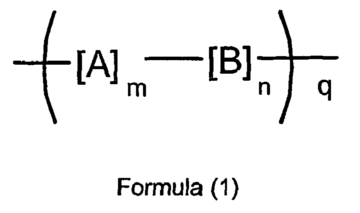
Wherein;
[A] is of Formula (9), 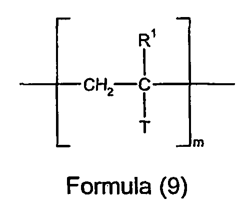
[B] is of Formula (10), 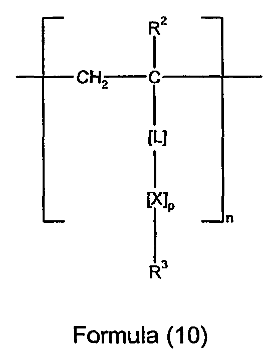 and [X] is of Formula (11), 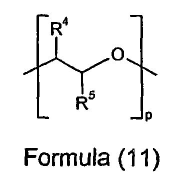
whereas [A] and [B] may occur in any order;
- T: is an optionally substituted substituent;
- L: is an optionally substituted linking group;
- R¹, R² and R³: are each independently H, optionally substituted C₁₋₂₀-alkyl or optionally substituted C₃₋₂₀-cycloalkyl;
- R⁴ and R⁵: are each independently H or C₁₋₄alkyl;
- q: is 15 to 1000;
- p: is 3 to 50; and
the molar ratio of [A] to [B] (m:n), is 1:10 to 10:1;
provided that R¹, R², R³, T and L do not contain an ionisable group and provided that at least one of R⁴ and R⁵ is H.
In the fifth aspect of the present invention preferences for [A], [B], m, n, q, T, L, X, R¹, R², R³, R⁴ and R⁵ are as hereinbefore defined with reference to the first aspect of the present invention.

The invention is further illustrated by the following examples in which all parts are by weight unless otherwise stated.

### Experimental Details.

### Example 1 - Preparation of Polymer 1 - A Non-Ionic Vinyl Copolymer.

A clean and dry two litre glass reactor was fitted with an overhead stirrer, nitrogen bleed, thermocouple and condenser. An initiator solution [1], was prepared by dissolving dimethyl 2,2' azobis isobutyrate (2.3g) (0.01 moles) in solvent (97.3g of a 50/50 w/w mixture of ethanol /distilled water). A monomer solution [2], was prepared containing solvent (490g of a 50/50 w/w mixture of ethanol/distilled water), methyl methacrylate (80g, 0.8 moles) and methoxy (polyethylene glycol 550) monomethacrylate (127g, 0.2 moles). Monomer solution [2] was added to the reactor along with additional solvent (270g of a 50/50 w/w mixture of ethanol/distilled water) to help wash the monomers into the reactor. The monomer solution [2] was washed into the reactor with additional solvent (100g of a 50/50 w/w mixture of ethanol/distilled water). The reactor was heated to 75°C using a Haake circulating water bath and then stirred at 180 rpm under a nitrogen blanket. At time zero initiator solution [1] (25g) was added to the reactor followed 30 minutes later by more initiator solution [1] (50g). The reactor contents were left for 3 hours 30 minutes before increasing the reactor temperature to 80°C. On reaching the required temperature additional initiator solution [1] (12.5 g) was added to the reactor which was then stirred for a further two hours after which time a final aliquot of initiator solution [1] (12.5g) was added. After two hours the polymeric solution was cooled and discharged from the reactor.

The total time of polymerisation was eight hours. The final solution was water white and free of particulate matter. The non-ionic co-polymer was formed in greater than 99% yield as determined by weight difference following exhaustive evaporation from a sample of the co-polymer solution.

The molecular weight of the co-polymer was determined using gel permeation chromatography (GPC) using polyethylene oxide as molecular weight standards. NMR Analysis was used to confirm the ratio of the repeat units [A], [B] and Dynamic Mechanical Thermal Analysis (DMTA) was used to determine the Tg of the co-polymer.

Co-polymers 2-8 in Table 1, containing different monomers [A] and [B] in various molar ratios, were prepared according to the same procedure as outlined above.

**Table 1. Composition of Non-Ionic Co-polymers.**

| Non-ionic Co-polymer | Monomer | | Length of PEG/PPG Units¹ | Molar Ratio of Repeat Units [A] : [B] | |
|---|---|---|---|---|---|
| | **[A]** **[B]** | | | | |
| 1 | MMA | PEG550Ma | 12 | 4 | 1 |
| 2 | MMA | PEG550Ma | 12 | 3 | 1 |
| 3 | MMA | PEG550Ma | 12 | 2 | 1 |
| 4 | EMA | PEG550Ma | 12 | 3 | 1 |
| 5 | i-BMA | PEG550Ma | 12 | 3 | 1 |
| 6 | BMA | PEG550Ma | 12 | 3 | 1 |
| 7 | i-Bornyl MA | PEG550Ma | 12 | 3 | 1 |
| 8 | MMA | PEG350Ma | 8 | 3 | 1 |
| 9 | MMA | PPG5MA | 5 | 4 | 1 |

The value¹ corresponds to p in Formulae (11) and (13)
- MMA: Methyl Methacrylate
- EMA: Ethyl Methacrylate
- i-BMA: iso Butyl Methacrylate
- BMA: Butyl Methacrylate
- i-Bornyl MA: iso Bornyl methacrylate
- PEG350Ma: Methoxy polyethylene glycol monomethacrylate with 7-8 ethylene oxide units.
- PEG550Ma: Methoxy polyethylene glycol monomethacrylate with 12-13 ethylene oxide units.
- PPG5MA: Methoxypolypropyleneglycol monomethacrylate with 5 propylene oxide units.

### Preparation of Non-ionic Co-polymer/Biocide Compositions.

Compositions 1 to 20 were prepared by mixing a 20% aqueous solution of poly (hexamethylenebiguanide) hydrochloride (PHMB) (5g), (available from Avecia Limited as Vantocil^{™}IB) to each of the polymers 1 to 8 from Table 1 as 20% solutions (water/ethanol 1/1) in varying quantities as set out in Table 2. The compositions were allowed to stand for 24 hours before being applied to substrates such as glass or ceramic tiles.

All of the compositions were low viscosity colourless transparent solutions, free from sediment and with excellent storage stability. Storage stability was tested by storing the compositions for 2 months at 52°C and was considered excellent if the viscosity of the composition remained unchanged and there was no formation of precipitate or gel particles.

Preparation of basic Copolymer compositions with various biocides looking at antifungal properties using polymer Example 4.

Compositions 21-28 were prepared by mixing with various biocides. To a sample of the polymer solution the biocide was added at concentrations ranging from 0.1 % - 0.5% wt/wt on total weight of the solution. The compositions were placed on a rotating mixer for 24 hours to form a homogenous composition and then applied to substrates such as glass or ceramic tile. The compositions were of low viscosity and free from sediment.

**Table 2. Compositions of the Non-ionic Co-polymer /PHMB Formulations.**

| Composition Number | Non-Ionic Co-polymer (Table 1) | Non-Ionic Co-polymer (wt%) | Polymeric biguanide (PHMB) other Biocide (wt%) |
|---|---|---|---|
| 1 | 1 | 37 | 63 |
| 2 | 1 | 50 | 50 |
| 3 | 1 | 55 | 45 |
| 4 | 1 | 71 | 29 |
| 5 | 1 | 74 | 26 |
| 6 | 1 | 80 | 20 |
| 7 | 1 | 83.3 | 16.7 |
| 8 | 1 | 85 | 15 |
| 9 | 1 | 95 | 5 |
| 10 | 2 | 83.3 | 16.7 |
| 11 | 2 | 95 | 5 |
| 12 | 3 | 83.3 | 16.7 |
| 13 | 3 | 95 | 5 |
| 14 | 4 | 83.3 | 16.7 |
| 15 | 4 | 95 | 5 |
| 16 | 5 | 95 | 5 |
| 17 | 6 | 95 | 5 |
| 18 | 7 | 95 | 5 |
| 19 | 8 | 95 | 5 |
| 20 | 9 | 90 | 10 |
| 21 | 4 | 99.9 | 0.1 of Biocide A |
| 22 | 4 | 99.8 | 0.2 of Biocide a |
| 23 | 4 | 99.8 | 0.2 of Biocide B |
| 24 | 4 | 99.5 | 0.5 of biocide B |
| 25 | 4 | 99.9 | 0.1 of Biocide C |
| 26 | 4 | 99.8 | 0.2 of Biocide C |
| 27 | 4 | 99.9 | 0.1 of biocide D |
| 28 | 4 | 99.8 | 0.2 of Biocide D |

| | | | |
|---|---|---|---|
| Biocide A n Butyl 1,2, benzisothiazolin Biocide B Dodecylethyldimethylammonium bromide Biocide C 3-iodopropargylbutvl carbamate Biocide D 2-octylisothiazolin-3-one Measurement of the Release of Biocidal Agent (PHMB) from the Non-ionic Co-polymer/PHMB compositions. | | | |

### Calibration of poly(hexamethylene biguanide) (PHMB) Concentration using UV Spectrometry.

Firstly the UV absorbance at 236nm of a known concentration of poly(hexamethylene biguanide) (PHMB) dissolved in water was measured (Perkin Elmer Lambda 900 UVNis/NIR Spectrophotometer). In a similar manner the UV absorbance at 236nm was measured for a series of samples prepared from known dilutions of the original PHMB aqueous solution. A calibration curve for PHMB concentration in aqueous solution was produced (Graph 1) by plotting UV absorbance against PHMB concentration.

In addition a similar UV calibration curve (Graph 1) was produced for PHMB in the presence of Polymer 1 Composition 9. Graph 1 illustrates that the non-ionic co-polymer did not significantly interfere with the determination of the PHMB concentration in the composition by this method.

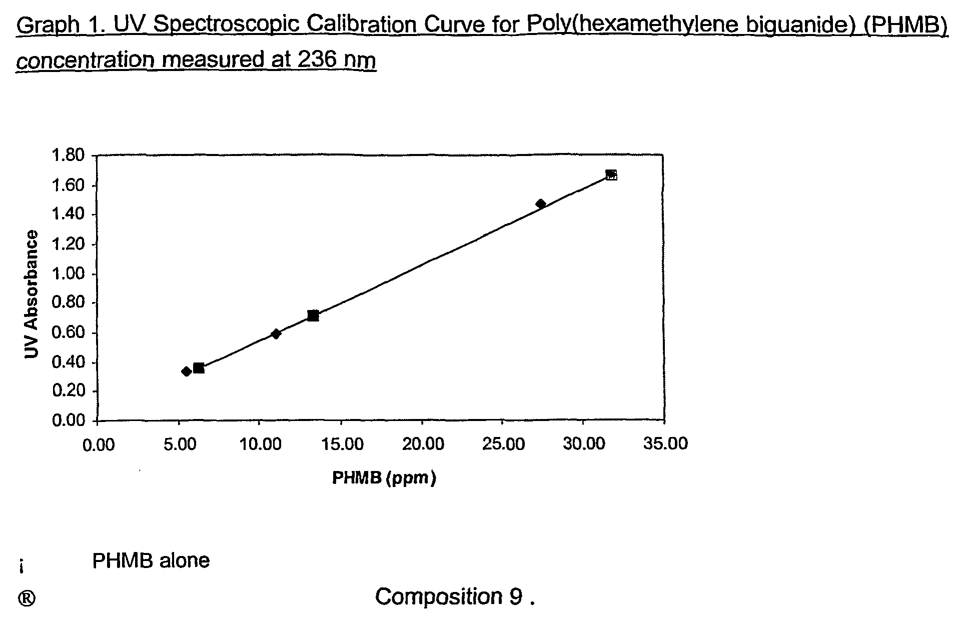

### General Method for the Measurement of the Rate of Poly(hexamethylene biguanide) (PHMB) Release from Films of Compositions 1 to 12 Using UV Analysis.

Compositions 1 to 20 were separately applied to clean glass panels (150mm x 100mm) and films were drawn down using a Sheen 250µ down bar. The films were allowed to dry and the coating weight noted.

Each coated glass panel was immersed separately in distilled water (1L) in a 2L beaker and stirred at a constant speed using a magnetic stirrer.

Samples (approximately 5cm³) were taken from the beaker in duplicate at regular intervals over the period of one hour. The samples were analysed using a UV spectrophotometer and the absorbance of each sample measured at a specific peak corresponding to the λ max of the poly(hexamethylene biguanide) (PHMB). The measured absorbance was directly related to the concentration of the poly(hexamethylene biguanide) (PHMB) in the solution.

### Measurement of the controlled release of poly(hexamethylene biguanide) (PHMB) from films of compositions 1 to 20

Using the methodology described above the following release profiles (Graphs 2 to 4) were generated: 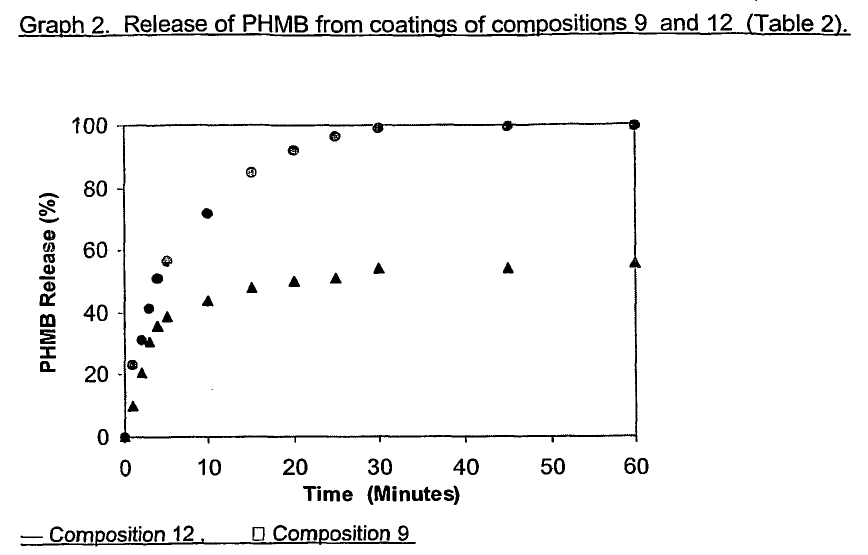 From Graph 2 the following can be concluded:
(i) Films of compositions 9 and 12 demonstrate a controlled release of PHMB.
(ii) The film of composition 9 releases approximately 50% of its PHMB over a 60 minute period.
(iii) The film of composition 12 releases 100% of its PHMB over a 30 minute period.
(iv) The film of composition 12 which contains a more hydrophilic polymer (Polymer 3) releases PHMB more rapidly. 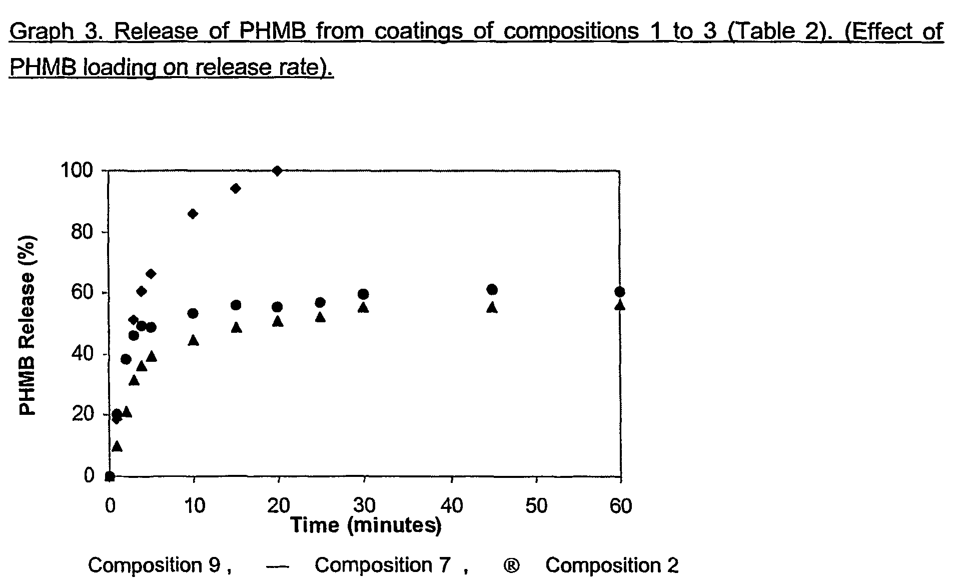
From Graph 3 the following can be concluded:
(i) Films of compositions 9 and 7, with 5 and 16.7% w/w PHMB respectively, release approximately 50% of their PHMB after 60 minutes.
(ii) A film of composition 2 with 50% w/w PHMB, released all of its PHMB after just 20 minutes.

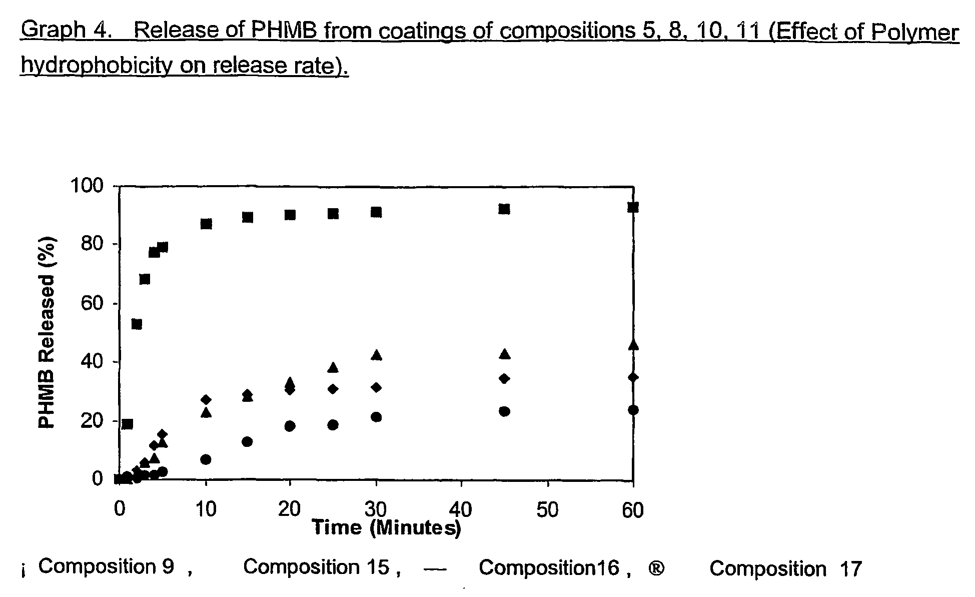

For ease of interpretation the polymer compositions used in the above formulations are more fully detailed in Table 4.

**Table 4**

| Composition Number | Polymer Number | Polymer Composition Molar Ratio [A]:[B] = 3:1 | |
|---|---|---|---|
| | | Monomer [A] Monomer [B] | |
| 9 | 2 | MMA | PEG550M |
| 15 | 4 | EMA | PEG550M |
| 16 | 5 | iBMA | PEG550Ma |
| 17 | 6 | BMA | PEG550Ma |

From Graph 4 the following can be concluded:
(i) PHMB is released most rapidly from the most hydrophilic film of Composition 9.
(ii) Replacing MMA, Polymer 2 (Composition 9) with EMA, Polymer 4 (Composition 15) significantly reduces the rate of PHMB release from greater than 80% to less than 50% after one hour, respectively.
(iii) In this test at 5% w/w PHMB loading, compositions 15 , 16 and 17 each control the release of PHMB for a period in excess of 1 hour.

Therefore, according to the present invention it was found that the rate of dissolution of poly(hexamethylene biguanide) from the non-ionic co-polymers could be controlled according to the co-polymer structure and by the ratio of non-ionic co-polymer to poly(hexamethylene biguanide). Moreover, the above illustrates that stable non-ionic co-polymer solutions in both water and water/ethanol mixture, can be prepared with polymeric biguanides such as poly(hexamethylene biguanide) hydrochloride.

### Calculation of Minimum Inhibitory Concentrations

The intrinsic antimicrobial activity of compositions of Polymer [1]¹ with various levels of PHMB were evaluated by measuring Minimum Inhibitory Concentrations (MICs).
1. Bacteria (*Pseudomonas aeruginosa* ATCC 15442) were grown on nutrient agar for 16 to 24 hours at 37°C (to give approx. 10⁹ cells per ml).
2. A 0.1 %(v/v) inoculum was used to seed fresh medium and 100 µl was then added to each well of a microtitre plate, except for the first well to which was added 200µl of inoculum.
3. Using doubling dilutions, the concentration of the PHMB non-ionic co-polymer formulations (Table 5) were varied in each well along the ordinate axis.
4. The presence or absence of growth was determined by visual inspection after 24 hours incubation at 37°C.
The MIC is the lowest concentration of the sample required to inhibit bacterial growth.

**Table 5: Intrinsic Antimicrobial Activity of Non-ionic Co-polymer [1]¹ with PHMB Formulations**

| Composition Number | % By Weight PHMB on Polymer [1]¹ | MIC versus *Pseudomonas aeruginosa* (ppm) |
|---|---|---|
| 1 | 63 | 92.5 |
| 3 | 45 | 13.5 |
| 4 | 29 | 9 |
| 5 | 26 | 10 |
| 6 | 20 | 12 |
| 8 | 15 | 12 |
| | 100 (Control) | 10 |

| | | |
|---|---|---|
| ¹ - for composition of Polymer [1] see Table 1. | | |

### Sustained Bactericidal Activity of Non-ionic Co-polymers with PHMB.

### Experimental Determination of the Residual Bactericidal Activity of Non-ionic Co-polymer/PHMB Compositions.

Non-ionic co-polymer/PHMB compositions were prepared as previously described (Table 2).

The residual antibacterial activity of the samples was evaluated by the following methodology;
1. All compositions were diluted to 0.5% active ingredient (a.i) PHMB. A 50µl aliquot of each sample was placed in a ceramic tile well and allowed to dry for approximately 1 hour).
2. Bacteria (*Ps.aeruginosa* ATCC 15442) were grown in nutrient broth at 37°C for approx. 16-20 hours.
3. An inoculum of approximately 10⁸ organisms per ml was prepared in physiological saline (0.85% NaCl).
4. A 150µl aliquot of bacterial inoculum was pipetted into the ceramic tile well, which had previously been coated by the PHMB/non-ionic co-polymer composition and incubated at room temperature.
5. After 5 minutes contact time the inoculum was removed by pipette and the number of surviving, viable organisms enumerated (samples were serially diluted in CEN neutraliser by 10², a 1 ml aliquot was added to 9ml of impedance broth and the RABIT^{™} was used to enumerate bacterial cells).
6. The PHMB/non-ionic co-polymer coated ceramic wells were then washed up to five times with 5ml aliquots of sterile distilled water.
7. Following each washing step, the samples were re-inoculated with a 150µl aliquot of bacterial inoculum.
8. As above the inoculum was removed after 5 minutes and the number of viable organisms enumerated by the method described above.

The RABIT^{™} (Rapid Automated Bacterial Impedance Technique) measures the change in conductance of a bacterial suspension over time. Actively growing bacteria break down uncharged or weakly charged molecules in a defined media to give end products that are highly charged. The resultant increase in conductance can be directly related to bacterial concentration by the use of a calibration curve. (For further background on this technique see: 'Technical Reference Paper - RAB-03, Don Whitley Scientific, 14 Otley Road, Shipley, West Yorkshire, UK, BD17 7SE).

Table 6 summarises the sustained bacterial activity of the non-ionic co-polymer/PHMB formulations obtained using the above techniques.

**Table 6. Sustained Bactericidal Activity of Non-ionic Co-polymer (Example 1) with PHMB.**

| Composition Number | % By Weight PHMB on Polymer [1]¹ | Log reduction versus *Ps.aeruginosa* after 5 minutes contact time | | |
|---|---|---|---|---|
| | | No washes 1 wash 2 washes | | |
| | 100 PHMB (Control) | 6.6 | 4.6 | 0.2 |
| 1 | 63 | 6.6 | 6.9 | 0.7 |
| 3 | 45 | 6.6 | 6.9 | 1 |
| 4 | 29 | 6.6 | 3.0 | 0.5 |
| 5 | 26 | 6.6 | 1.8 | 0.5 |
| 6 | 20 | 6.6 | 1.4 | 0.5 |
| 8 | 15 | 6.6 | 1.0 | 0.5 |

Table 6 demonstrates the sustained effect of poly(hexamethylene biguanide)(PHMB)/non-ionic co-polymer compositions. The control sample (with PHMB alone) achieved a log 6.6 reduction in bacteria value initially, log 4.6 reduction after one wash and log 0.2 after 2 washes. Whilst this data demonstrates the effectiveness of PHMB in disinfection applications (equivalent to zero washes data), sustained effect behaviour is demonstrated by the increased log kill values after 1 and 2 washes.

For example, composition 3 Co-polymer [1] achieved log 6.6, log 6.9 and log 1 reductions after 0, 1 and 2 washes respectively. For this particular non-ionic co-polymer 45% PHMB is close to optimal in achieving a sustained antimicrobial effect. Other PHMB/non-ionic co-polymer combinations have maximum sustained effect values at different ratios.

**Table 7: Sustained Bactericidal Activity of Non-ionic Co-polymer (Example 3 & 9) with PHMB.**

| Composition Number | % By Weight PHMB on Polymer | Log reduction versus. *Ps.aeruginosa* after 5 minutes contact time. | | |
|---|---|---|---|---|
| | | No washes 1 wash 2 washes | | |
| | 100 PHMB (Control) | 6.6 | 4.6 | 0.2 |
| 12 | 16.7 | 6.9 | 6.6 | 0.8 |
| 13 | 5 | 6.9 | 8.9 | 2.2 |
| 20 | 10 | 1.6 | 2.5 | 0.5 |

Table 7 illustrates that for a composition containing PHMB , sustained bactericidal activity is maximised in composition 13 for which log reductions of 6.9, 8.9 and 2.2 are achieved after 0, 1 and 2 washes respectively. This formulation demonstrates improved sustained biocidal effect over both the control (PHMB alone which gives log reductions of 6.6, 4.6 and 0.2 after 0, 1 and 2 washes respectively) and the PHMB/Polymer [1] formulations (Table 6).

### Sustained Fungicidal Activity of Non-ionic co-polymers with Various Biocides

### Experimental determination of the Residual Fungicidal Activity of Non-ionic co-polymer/Biocide Formulations.

Non-ionic co-polymer/Biocide compositions were prepared as previously described (Table 2).

The residual antifungal activity of the samples was determined by the following methodology:
1. Films of each composition were created on glass microscope slides using a '0' K-Bar and allowed to dry for no less than 24 hours.
2. Fungi (*Aspergillus niger* ATCC 16404) were grown on malt agar plates at 25°C for approx. 7 days.
3. An inoculum of approximately 10⁷ spores per ml was prepared in physiological saline (0.85% NaCl).
4. A 150µl aliquot of fungal inoculum was added to the surface of the compositions and incubated at room temperature for 24 hours.
5. The number of surviving, viable organisms were then enumerated (samples were washed into a neutralising medium, serially diluted in physiological saline and plated out onto malt agar).
6. Each composition was then washed ten times by spraying with sterile distilled water.
7. Each composition was then re-inoculated and after 24 hours the number of viable organisms enumerated by the method described above.

Table 8 summarises the sustained fungicidal activity of the Non-ionic co-polymer/Biocide formulations obtained using the above technique.

**Table 8: Sustained Fungicidal Activity of Non-ionic Co-polymers (Example 4) with Various Biocides.**

| Composition Number | Biocide | Weight Ratio (w/w) Biocide: polymer | log reduction vs. *A.niger* @ 24h after no. of washes; | |
|---|---|---|---|---|
| | | | 0 | |
| | | | 10 | |
| 22 | Biocide A | 499:1 | 1.7 | 0.2 |
| 24 | Biocide B | 199:1 | 3.4 | 3.1 |
| 26 | Biocide C | 499:1 | 3.4 | 3.4 |
| 28 | Biocide D | 499:1 | 3.4 | 3.4 |

It can be concluded that not only can stable formulations be prepared with the various biocides but that a sustained effect could be maintained using the spray washing protocol.

The results in Table 8 show that 3 formulations gave excellent sustained fungicidal activity.

## Claims

1. A composition comprising:
(i) an anti-microbial agent comprising a polymeric biguanide, alone or in combination with at least one other microbiologically active component selected from the group consisting of quaternary ammonium compounds, monoquaternary heterocyclic amine salts, urea derivatives, amino compounds, imidazole derivatives, nitrile compounds, tin compounds or complexes, isothiazolin-3-ones, thiazole derivatives, nitro compounds, iodine compounds, aldehyde release agents, thiones, triazine derivatives, oxazolidine and derivatives thereof, furan and derivatives thereof, carboxylic acids and the salts and esters thereof, phenol and derivatives thereof, sulphone derivatives, imides, thioamides, 2-mercapto-pyridine-N-oxide, azole fungicides, strobilurins, amides, carbamates, pyridine derivatives, compounds with active halogen groups, and organometallic compounds ; and
(ii) a non-ionic co-polymer of Formula (1) 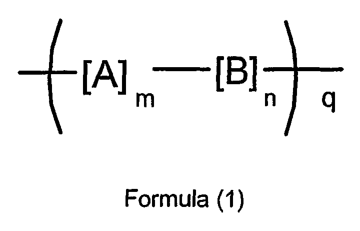
wherein:
[A] is of Formula (9), 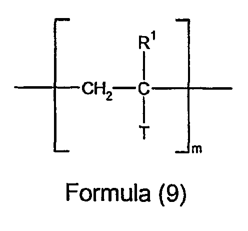
[B] is of Formula (10), 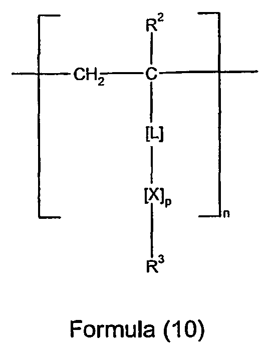 and [X] is of Formula (11), 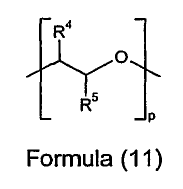
wherein [A] and [B] may occur in any order;
T is an optionally substituted substituent;
L is an optionally substituted linking group;
R¹, R² and R³ are each independently H, optionally substituted C₁₋₂₀-alkyl or optionally substituted C₃₋₂₀-cycloalkyl;
R⁴ and R⁵ are each independently H or C₁₋₄alkyl;
q is 15 to 1000;
p is 3 to 50; and
the molar ratio of [A] to [B] (m:n), is 1:10 to 10:1;
provided that R¹, R², R³, T and L do not contain an ionisable group and provided that at least one of R⁴ and R⁵ is H.

2. A composition according to claim 1 wherein the anti-microbial agent comprises a linear polymeric biguanide.

3. A composition according to claim 1 or claim 2 wherein the polymeric biguanide is a linear polymeric biguanide comprising a mixture of polymer chains in which the individual polymer claims excluding the terminating groups contain at least one recurring unit having two biguanide groups of Formula (4): 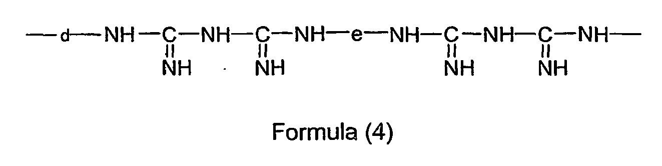 wherein d and e represent bridging groups which may be the same or different and in which together the total of the number of carbon atoms directly interposed between the pairs of nitrogen atoms linked by d plus the number of carbon atoms directly interposed between the pairs of nitrogen atoms linked by e is more than 9 and less than 17.

4. A composition according to any one of the preceding claims wherein the polymeric biguanide is of Formula (6): 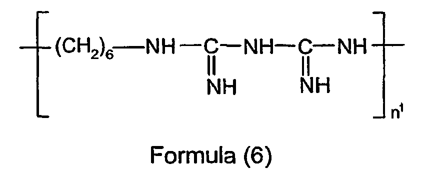 wherein n¹ is from 4 to 20.

5. A composition according to any one of the preceding claims wherein the non-ionic co-polymer comprises a cloud point of greater than 15°C, preferably greater than 25°C.

6. A composition according to any one of the preceding claims wherein the non-ionic co-polymer comprises from 10 to 90% by weight of [B] and from 10 to 90% by weight of [A].

7. A composition according to any one of the preceding claims wherein R¹, R² and R³ are each independently H or -CH₃.

8. A composition according to any one of the preceding claims wherein R⁴ and R⁵ are each independently H.

9. A composition according to any one of the preceding claims wherein T comprises a group of the Formula -C(O)OR⁶, wherein R⁶ comprises C₁₋₁₀-alkyl; and L comprises a group of the Formula,

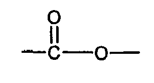

10. A composition according to any one of the preceding claims wherein the non-ionic co-polymer of Formula (1) comprises [A] of Formula (12), 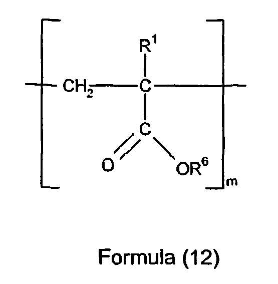 and [B] of Formula (13), 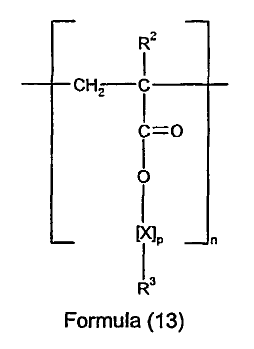 wherein:
R¹, R² and R³ is each independently H or CH₃;
R⁶ is C₁₋₁₀alkyl, optionally substituted by a ketone, ether, -OH, epoxide, silane or keto ester group; and
n, m and p and X are as defined with reference to claims 1 to 9.

11. A composition according to any one of the preceding claims wherein the weight ratio of polymeric biguanide to non-ionic co-polymer is from 100:1 to 1:1000 weight percent, more preferably from 20:1 to 1:500 weight percent.

12. A composition according to any one of the preceding claims which comprises a pH of from 1 to 12, preferably from 3 to 9.

13. A composition according to claim 1 wherein the anti-microbial agent comprises a fungicide.

14. A formulation comprising:
(i) a linear polymeric biguanide;
(ii) a non-ionic co-polymer; and
(iii) a carrier,
wherein the polymeric biguanide and non-ionic co-polymer are as defined in any one of claims 1 to 12.

15. A formulation according to claim 14 wherein the carrier is water or a mixture of water and/or a water miscible organic solvent.

16. A formulation according to claim 14 or 15 which comprises from 0.01 to 5% by weight polymeric biguanide and from 0.01 to 50% by weight non-ionic co-polymer.

17. A formulation according to any one of claims 14 to 16 which comprises a pH in the range of from 1 to 12, preferably of from 3 to 9.

18. A non-therapeutic method of reducing the level of micro-organisms on a surface which comprises contacting the surface with a composition as claimed in any one of claims 1 to 13 or a formulation as claimed in claims 14 to 17.

## Patentansprüche

1. Zusammensetzung, umfassend:
(i) einen antimikrobiellen Wirkstoff, umfassend ein polymeres Biguanid, allein oder in Kombination mit mindestens einer anderen mikrobiologisch wirksamen Komponente, die aus der Gruppe, die aus quaternären Ammoniumverbindungen, mono-quaternären heterozyklischen Aminsalzen, Harnstoffderivaten, Aminoverbindungen, Imidazolderivaten, Nitrilverbindungen, Zinnverbindungen oder - komplexen, Isothiazolin-3-onen, Thiazolderivaten, Nitroverbindungen, Jodverbindungen, Aldehydfreisetzenden Wirkstoffen, Thionen, Triazinderivaten, Oxazolidin und Derivaten desselben, Furan und Derivaten desselben, Carbonsäuren und den Salzen und Estern derselben, Phenol und Derivaten desselben, Sulfonderivaten, Imiden, Thioamiden, 2-Mercapto-pyridin-N-oxid, Azol-Fungiziden, Strobilurinen, Amiden, Carbamaten, Pyridinderivaten, Verbindungen mit aktiven Halogengruppen und organometallischen Verbindungen besteht, ausgewählt ist, und
(ii) ein nicht-ionisches Copolymer mit der Formel (1) 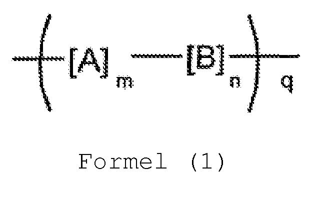
wobei:
[A] die Formel (9) aufweist, 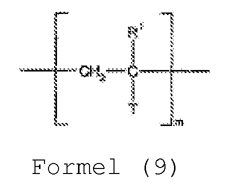
[B] die Formel (10) aufweist, 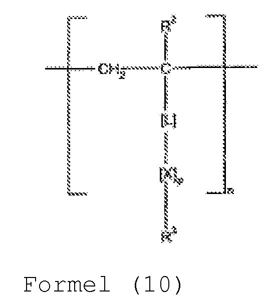 und [X] die Formel (11) aufweist, 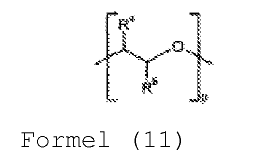
wobei [A] und [B] in beliebiger Reihenfolge vorhanden sein können,
T ein wahlweise substituierter Substituent ist,
L eine wahlweise substituierte verbindende Gruppe ist,
R¹, R² und R³ jeweils unabhängig voneinander H, wahlweise substituiertes C₁₋₂₀-Alkyl oder wahlweise substituiertes C₃₋₂₀-Cycloalkyl sind,
R⁴ und R⁵ jeweils unabhängig voneinander H oder C₁₋₄-Alkyl sind,
q gleich 15 bis 1000 ist,
p gleich 3 bis 50 ist, und
das Molverhältnis von [A] zu [B] (m:n) 1:10 bis 10:1 beträgt,
sofern R¹, R², R³, T und L keine ionisierbare Gruppe enthalten und
sofern mindestens einer der Reste R⁴ und R⁵ H ist.

2. Zusammensetzung nach Anspruch 1, wobei der antimikrobielle Wirkstoff ein lineares polymeres Biguanid umfasst.

3. Zusammensetzung nach Anspruch 1 oder 2, wobei das polymere Biguanid ein lineares polymeres Biguanid ist, das eine Mischung von Polymerketten umfasst, bei der die individuellen Polymerketten mit Ausnahme der terminierenden Gruppen mindestens eine sich wiederholende Einheit, die zwei Biguanidgruppen mit der Formel (4) besitzt, enthalten: 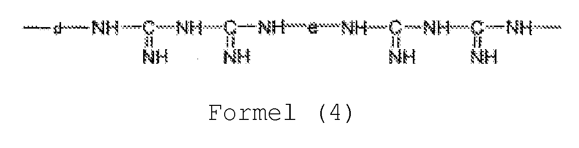 wobei d und e überbrückende Gruppen darstellen, die gleich oder unterschiedlich sein können und in denen, zusammengenommen, die Gesamtzahl der Anzahl von Kohlenstoffatomen, die direkt zwischen den durch d verbundenen Paaren von Stickstoffatomen vorhanden sind plus die Anzahl der Kohlenstoffatome, die direkt zwischen den durch e verbundenen Paaren von Stickstoffatomen vorhanden sind, größer als 9 und kleiner als 17 ist.

4. Zusammensetzung nach einem der vorstehenden Ansprüche, wobei das polymere Biguanid die Formel (6) besitzt: 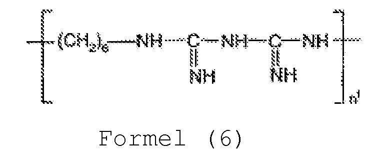 wobei n¹ von 4 bis 20 beträgt.

5. Zusammensetzung nach einem der vorstehenden Ansprüche, wobei das nicht-ionische Copolymer einen Trübungspunkt über 15°C, vorzugsweise über 25°C, besitzt.

6. Zusammensetzung nach einem der vorstehenden Ansprüche, wobei das nicht-ionische Copolymer von 10 bis 90 Gewichtsprozent [B] und von 10 bis 90 Gewichtsprozent [A] umfasst.

7. Zusammensetzung nach einem der vorstehenden Ansprüche, wobei R¹, R² und R³ jeweils unabhängig voneinander H oder -CH₃ sind.

8. Zusammensetzung nach einem der vorstehenden Ansprüche, wobei R⁴ und R⁵ jeweils unabhängig voneinander H sind.

9. Zusammensetzung nach einem der vorstehenden Ansprüche, wobei T eine Gruppe der Formel -C(O)OR⁶ umfasst, wobei R⁶ C₁₋₁₀-Alkyl umfasst und L eine Gruppe mit der Formel 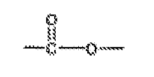 umfasst.

10. Zusammensetzung nach einem der vorstehenden Ansprüche, wobei das nicht-ionische Copolymer mit der Formel (1) [A] mit der Formel (12) 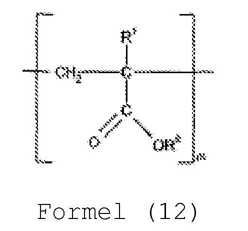 und [B] mit der Formel (13) 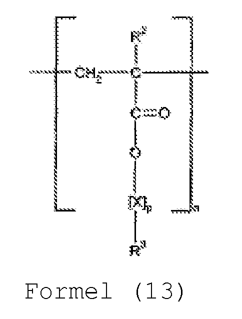 umfasst, wobei
R¹, R² und R³ jeweils unabhängig voneinander H oder CH₃ sind, R⁶ C₁₋₁₀-Alkyl, wahlweise mit einer Keton-, Ether-, -OH, Epoxid-, Silan- oder Ketoestergruppe substituiert, ist
und
n, m und p und X unter Bezugnahme auf die Ansprüche 1 bis 9 festgelegt sind.

11. Zusammensetzung nach einem der vorstehenden Ansprüche, wobei das Gewichtsverhältnis von polymerem Biguanid zu nichtionischem Copolymer von 100:1 bis 1:1000 Gewichtsprozent, bevorzugter von 20:1 bis 1:500 Gewichtsprozent, beträgt.

12. Zusammensetzung nach einem der vorstehenden Ansprüche, die einen pH-Wert von 1 bis 12, bevorzugt von 3 bis 9, aufweist.

13. Zusammensetzung nach Anspruch 1, wobei der antimikrobielle Wirkstoff ein Fungizid umfasst.

14. Formulierung, umfassend
(i) ein lineares polymeres Biguanid,
(ii) ein nicht-ionisches Copolymer und
(iii) einen Träger,
wobei das polymere Biguanid und das nicht-ionische Copolymer wie in einem der Ansprüche 1 bis 12 definiert sind.

15. Formulierung nach Anspruch 14, wobei der Träger Wasser oder eine Mischung aus Wasser und/oder einem mit Wasser mischbaren organischen Lösungsmittel ist.

16. Formulierung nach Anspruch 14 oder 15, die von 0,01 bis 5 Gewichtsprozent polymeres Biguanid und von 0,01 bis 50 Gewichtsprozent nicht-ionisches Copolymer umfasst.

17. Formulierung nach einem der Ansprüche 14 bis 16, die einen pH-Wert im Bereich von 1 bis 12, bevorzugt von 3 bis 9, aufweist.

18. Nicht-therapeutisches Verfahren zur Reduzierung der Menge von Mikroorganismen auf einer Oberfläche, das das Inkontaktbringen der Oberfläche mit einer Zusammensetzung, wie sie in einem der Ansprüche 1 bis 13 beansprucht wird, oder einer Formulierung, wie sie in den Ansprüchen 14 bis 17 beansprucht wird, umfasst.

## Revendications

1. Composition comprenant :
(i) un agent antimicrobien comprenant un biguanide polymère, seul ou en combinaison avec au moins un autre composant microbiologiquement actif choisi dans le groupe constitué par des composés d'ammonium quaternaire, des sels d'amine hétérocyclique monoquaternaire, des dérivés d'urée, des composés aminés, des dérivés d'imidazole, des composés de nitrile, des composés ou des complexes d'étain, des isothiazolin-3-ones, des dérivés de thiazole, des composés nitrés, des composés de l'iode, des agents de libération d'aldéhyde, des thiones, des dérivés de triazine, l'oxazolidine et des dérivés de celle-ci, le furanne et des dérivés de celui-ci, des acides carboxyliques et les sels et esters de ceux-ci, le phénol et des dérivés de celui-ci, des dérivés de sulfone, des imides, des thio-amides, le 2-mercapto-pyridine-N-oxyde, des fongicides d'azole, des strobilurines, des amides, des carbamates, des dérivés de pyridine, des composés avec des groupes halogénés actifs, et des composés organométalliques , et
(ii) un copolymère non ionique de formule 1 : 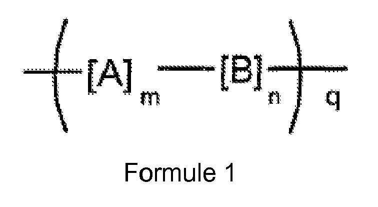
dans laquelle :
[A] est de formule 9 : 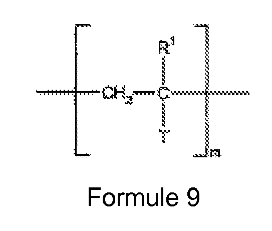
[B] est de formule 10 : 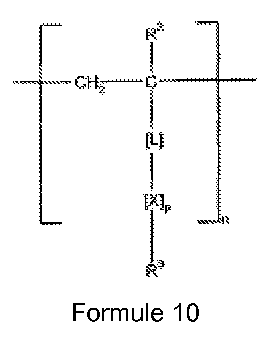 et [X] est de formule 11 : 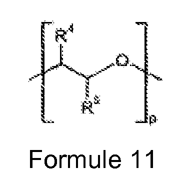
dans laquelle [A] et [B] peuvent se trouver dans un ordre quelconque ;
T est un substituant éventuellement substitué ;
L est un groupe de liaison éventuellement substitué ;
R¹, R² et R³ sont chacun indépendamment H, un groupe alkyle en C₁₋₂₀ éventuellement substitué ou un groupe cycloalkyle en C₃₋₂₀ éventuellement substitué ;
R⁴ et R⁵ sont chacun indépendamment H ou un groupe alkyle en C₁₋₄ ;
q vaut de 15 à 1 000 ;
p vaut de 3 à 50; et
le rapport molaire de [A] à [B] (m/n), est de 1/10 à 10/1 ;
à condition que R¹, R², R³, T et L ne contiennent pas un groupe ionisable et à condition qu'au moins l'un de R⁴ et R⁵ soit H.

2. Composition selon la revendication 1, dans laquelle l'agent antimicrobien comprend un biguanide polymère linéaire.

3. Composition selon la revendication 1 ou la revendication 2, dans laquelle le biguanide polymère est un biguanide polymère linéaire comprenant un mélange de chaînes polymères dans lequel les chaînes polymères individuelles à l'exclusion des groupes terminaux contiennent au moins une unité récurrente ayant deux groupes biguanide de formule 4 : 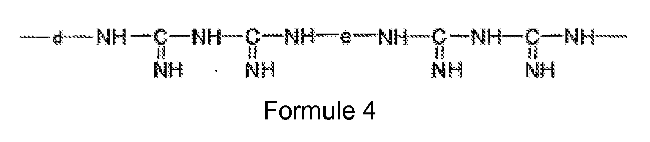 dans laquelle d et e représentent des groupes de pontage qui peuvent être les mêmes ou différents et dans lequel le total général du nombre d'atomes de carbone directement interposés entre les paires d'atomes d'azote liées par d plus le nombre d'atomes de carbone directement interposés entre les paires d'atomes d'azote liées par e est supérieur à 9 et inférieur à 17.

4. Composition selon l'une quelconque des revendications précédentes, dans laquelle le biguanide polymère est de formule 6 : 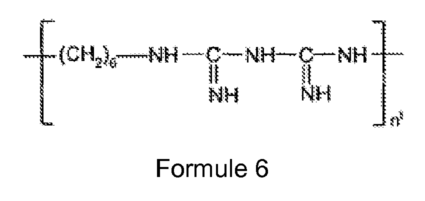 dans laquelle n¹ vaut de 4 à 20.

5. Composition selon l'une quelconque des revendications précédentes, dans laquelle le copolymère non ionique comprend un point de trouble de plus de 15 °C, de préférence de plus de 25 °C.

6. Composition selon l'une quelconque des revendications précédentes, dans laquelle le copolymère non ionique comprend de 10 à 90 % en poids de [B] et de 10 à 90 % en poids de [A].

7. Composition selon l'une quelconque des revendications précédentes, dans laquelle R¹, R² et R³ sont chacun indépendamment H ou -CH₃.

8. Composition selon l'une quelconque des revendications précédentes, dans laquelle R⁴ et R⁵ sont chacun indépendamment H.

9. Composition selon l'une quelconque des revendications précédentes, dans laquelle T comprend un groupe de formule -C(O)OR⁶, dans laquelle R⁶ comprend un groupe alkyle en C₁₋₁₀ ; et L comprend un groupe de formule :

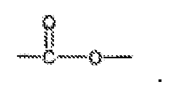

10. Composition selon l'une quelconque des revendications précédentes, dans laquelle le copolymère non ionique de formule 1 comprend [A] de formule 12 : 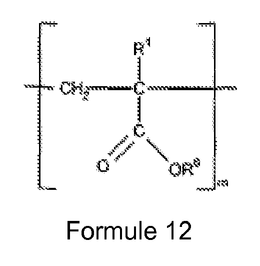 et [B] de formule 13 : 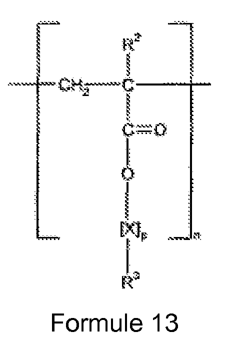 dans laquelle :
R¹, R² et R³ sont chacun indépendamment H ou -CH₃ ;
R⁶ est un groupe alkyle en C₁₋₁₀, éventuellement substitué par un groupe cétone, éther, -OH, époxyde, silane ou cétoester ;
et
n, m et p et X sont tels que définis en référence aux revendications 1 à 9.

11. Composition selon l'une quelconque des revendications précédentes, dans laquelle le rapport en poids du biguanide polymère au copolymère non ionique est de 100:1 à 1:1000 % en poids, de manière davantage préférée, de 20:1 à 1:500 % en poids.

12. Composition selon l'une quelconque des revendications précédentes, qui comprend un pH allant de 1 à 12, allant de préférence de 3 à 9.

13. Composition selon la revendication 1, dans laquelle l'agent antimicrobien comprend un fongicide.

14. Formulation comprenant :
(i) un biguanide polymère linéaire ;
(ii) un copolymère non ionique ; et
(iii) un support,
dans laquelle le biguanide polymère et le copolymère non ionique sont tels que définis dans l'une quelconque des revendications 1 à 12.

15. Formulation selon la revendication 14, dans laquelle le support est l'eau ou un mélange d'eau et/ou d'un solvant organique miscible à l'eau.

16. Formulation selon les revendications 14 ou 15, qui comprend de 0,01 à 5 % en poids de biguanide polymère et de 0,01 à 50 % en poids de copolymère non ionique.

17. Composition selon l'une quelconque des revendications 14 à 16, qui comprend un pH dans la plage allant de 1 à 12, allant de préférence de 3 à 9.

18. Procédé non thérapeutique réduisant le niveau de microorganismes sur une surface qui comprend la mise en contact de la surface avec une composition selon l'une quelconque des revendications 1 à 13 ou une formulation selon les revendications 14 à 17.
